# EUROPEAN PATENT APPLICATION

(11) **EP 2 803 356 A1**
(43) Date of publication of application: **19.11.2014**
(21) Application number: 14166640.4
(22) Date of filing: 31.03.2009
(51) Int. Cl.: A61K 31/4164, C07D 233/06, C07D 249/16, A61K 31/4184, C07D 233/58, C07D 401/04, A61K 31/4196, C07D 233/60, C07D 401/14, A61P 25/16, C07D 233/61, A61P 25/18, C07D 235/20

(54) **Method for treating neurological disorders with imidazolium and imidazolinium compounds**

(30) Priority: 31.03.2008 US 64870 P
(62) Divisional of application: 09727187.8
(71) Applicant: Agency for Science, Technology and Research, Singapore 138632 (SG)
(72) Inventor: Zhuo, Lang, 138669 Singapore (SG); Ho, Gideon, 138669 Singapore (SG); Zhang, Yugen, 138669 Singapore (SG); Ying, Jackie Y., 138669 Singapore (SG)
(74) Representative: Andrews, Robert

(57) **Abstract**

There is presently provided methods for delivering a neuroprotective agent to a neural cell. The methods comprise contacting a neural cell with an imidazolium or imidazolinium compound as described herein, including an imidazolium or imidazolinium salt.

## Description

### CROSS-REFERENCE TO RELATED APPLICATION

This application claims benefit of, and priority from, U.S. provisional patent application No. 61/064,870 filed on March 31,2008, the contents of which are fully incorporated herein by reference.

### FIELD OF THE INVENTION

The present invention relates generally to methods and uses of a compound for delivering a neuroprotective agent to a neural cell, including methods and uses of a compound for treating neurological disorders.

### BACKGROUND OF THE INVENTION

The nervous system includes two major types of neural cells: neurons which are the major functional cells in the nervous system and carry out the fundamental tasks of receiving, conducting and transmitting signals and glial cells which provide physical and nutritional support to neurons. Neurons and glial cells have been implicated in many diseases and disorders including neurological disorders such as Parkinson's disease, Alzheimer's disease and multiple sclerosis. Neurological disorders can have devastating effects on the individual and result in high social costs associated with chronic care and lost productivity. Thus, there is a need to develop effective treatment and one of the main goals of therapy for neurological disorders is the therapeutic protection of neural cells.

Common features of many neurological disorders include loss of neurons and gliosis. Gliosis is the proliferation of glial cells in response to damage to the central nervous system, usually resulting in the formation of a glial scar that may inhibit proper functioning of the central nervous system.

Damage associated with neurological disorders may occur throughout the central nervous system including the substantia nigra pars compacta (SNpc) and striatum regions of the brain. In addition, cells of the peripheral nervous system may also be affected by neurological disorders.

Furthermore, neurological disorders have been associated with the occurrence of neurodegeneration and gliosis in the eye. The retina and optic nerve originate as embryonic outgrowths from the brain and thus are extensions of the central nervous system. Thus the retina may also be afflicted in neurodegenerative diseases or neurological disorders. For example, Parkinson's disease is associated with neurodegeneration of the visual system and considerable psychophysical and electrophysiological evidence suggests that visual dysfunction in Parkinson's disease patients results from dopaminergic (DA) deficiency, possibly in the retina. (Bodi-Wollner, 1990; Peters et al., 2000). Furthermore, retinopathy, a non-inflammatory degenerative disease of the retina that leads to visual field loss or blindness, has been associated with neurotoxicity and neurodegenerative diseases.

The etiology of neurological disorders such as Parkinson's disease, Alzheimer's disease and multiple sclerosis is not entirely clear. However, it is thought that these disorders have both genetic and environmental origins. Furthermore, oxidative stress by the reactive oxygen species (ROS) is believed to play an important role in the pathogenesis of neurological disorders including Parkinson's disease.

Parkinson's disease is the second most common neurodegenerative disorder, just trailing Alzheimer's disease, worldwide. Parkinson's disease is characterized by symptoms of tremor at rest, slowness of voluntary movements, rigidity, and postural instability, and is attributed primarily to the loss of neurons of the nigrostriatal DA pathway, resulting in a deficit in the brain DA level. (Marin et al., 2005). Ongoing oxidative stress and inflammatory processes have been observed particularly in the SNpc of Parkinsonian brains and in the animal models of Parkinson's disease (Olanow and Tatton, 1999; Dauer and Przedborski, 2003; Hirsch et al., 2005; Bezard et al., 2006). This includes alterations in the activities of the antioxidative enzymes, superoxide dismutase (SOD) and catalase (Peng et al., 2005), a decrease in the level of reduced glutathione (GSH) (Grunblatt et al., 2001), a proliferation of reactive microglia with a highly significant increase in many cytokines, including tumor necrosis factor-α (TNF-α), interleukin-1 (IL-1) and IL-2 (Mogi et al., 1994a; Mogi et al., 1994b), along with an increase in the level of iron within the reactive microglia and the melanin containing DA neurons (Jellinger, 1999).

In recognition of the oxidation theory of Parkinson's disease etiology, anti-oxidative (Levites et al., 2001; Choi et al., 2002; Cleren et al., 2005) and antiinflammatory (Wu et al., 2002; Hirsch et al., 2005) approaches have been devised as therapeutic strategies to combat Parkinson's disease in animals induced with MPTP (1-methyl-4-phenyl-1,2,3,6-tetrahydropyridine). MPTP has been well documented to induce the symptoms of Parkinson's disease including degeneration of DA neurons in the SNpc (Chen et al., 2003), mitochondrial dysfunction, production of oxidative stress, release of proinflammatory cytokines and gliosis (Smeyne and Jackson, 2005). Evidence from an MPTP induced animal model of Parkinson's disease showed that natural anti-oxidants, such as (-)-epigallocatechin gallate (EGCG) from green tea, can attenuate Parkinson's disease progression (Levites et al., 2001).

Several approved drugs currently in clinical use are reported to attenuate and slow down some aspects of Parkinson's disease. However, there is no drug available on the market that can effectively reverse the symptoms of Parkinson's disease, or stop the progression of the disease. Furthermore, all of the currently available drugs show significant adverse effects, including toxicity and psychiatric disorders. Thus, there is a great need for better medicines to treat Parkinson's disease and other neurological disorders.

### SUMMARY OF INVENTION

There is presently provided methods for delivering a neuroprotective agent to a neural cell, the method comprising contacting a neural cell with imidazolium and imidazolinium compounds, including imidazolium and imidazolinium salts, as described herein.

There is also presently provided the use of imidazolium and imidazolinium compounds for delivering a neuroprotective agent to a neural cell or in the manufacture of a medicament for delivering a neuroprotective agent to a neural cell.

In addition, the methods and uses presently provided may be used for the treatment of neurological disorders.

In one aspect there is provided, a method for delivering a neuroprotective agent to a neural cell, the method comprising contacting the neural cell with a compound of general formula I or an oligomer or polymer thereof: 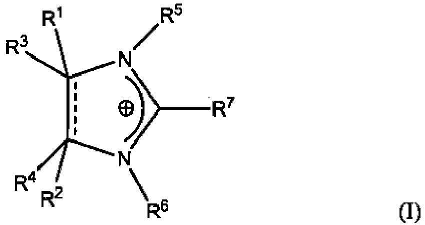 wherein:
the dashed line is absent or is present as a bond to form a second bond between the carbon to which R¹ and R³ are attached and the carbon to which R² and R⁴ are attached;
R¹ and R²:
(i) are each independently H, straight or branched C₁-C₆ alkyl, straight or branched C₂-C₆ alkenyl, straight or branched C₂-C₆ alkynyl, C₆-C₁₀ aryl;
(ii) together with their central ring atoms form a 6- to 10-membered fused saturated, unsaturated or aromatic ring system;
(iii) R¹ and R⁵ together with their central ring atoms, or R² and R⁶ together with their central ring atoms, form a 5- to 10-membered fused saturated, unsaturated or aromatic ring system and the other of R¹ and R² is as defined above in (i); or
(iv) R¹ and R⁵ together with their central ring atoms and R² and R⁶ together with their central ring atoms each independently form a 5- to 10-membered fused saturated, unsaturated or aromatic ring system;
R³ and R⁴ are both H, or, when R¹ and R² together with their central ring atoms form a 6- to 10-membered fused aromatic ring system or when the dashed line is present as a bond, R³ and R⁴ are absent;
R⁵ or R⁶:
(i) are as defined above for R¹ and R²; or
(ii) are each independently straight or branched C₁-C₆ alkyl, straight or branched C₂-C₆ alkenyl, straight or branched C₂-C₆ alkynyl, C₃-C₁₈ cycloalkyl including fused cycloalkyl ring systems, C₆-C₁₀ aryl, C₆-C₁₀ aryl-C₁-C₆ alkyl, C₆-C₁₀ aryl-C₂-C₆ alkenyl, or C₆-C₁₀ aryl-C₂-C₆ alkynyl, C₁-C₆ alkyl-C₆-C₁₀ aryl, C₂-C₆ alkenyl-C₆-C₁₀ aryl, or C₂-C₆ alkynyl-C₆-C₁₀ aryl;
R⁷ is H, C₁-C₆ alkyl or C₆-C₁₀ aryl;
in which any of R¹ to R⁶, where applicable, optionally has one or more carbon atoms replaced with a heteroatom selected from N, O, S and P and is optionally substituted with one or more of straight or branched C₁-C₆ alkyl, straight or branched C₂-C₆ alkenyl, straight or branched C₂-C₆ alkynyl, C₃-C₁₈ cycloalkyl including fused cycloalkyl ring systems, C₆-C₁₀ aryl, fluoro, tri-fluoro-methyl, cyanato, isocyanato, carboxyl, C₁-C₆ acyloxy, C₁-C₆ acyl, carbonyl, amino, acetyl, acetoxy, oxo, nitro, hydroxyl, C₁-C₆ alkylcarboxy, C₁-C₆ alkoxy, C₂-C₆ alkenoxy, C₂-C₆ alkynoxy; and
in which one of the central ring carbon atom to which R¹ and R³ are attached and the central ring carbon to R² and R⁴ are attached is optionally replaced with a nitrogen atom;
or a pharmaceutically acceptable salt of the compound or of the oligomer or polymer of the compound.

In another aspect, there is provided use of a compound for delivering a neuroprotective agent to a neural cell, the compound having a structure of general formula I or an oligomer or polymer thereof.

In another aspect, there is provided use of a compound in the manufacture of a medicament for delivering a neuroprotective agent to a neural cell, the compound having a structure of general formula I or an oligomer or polymer thereof.

In particular embodiments, the compound is an imidazolium or an imidazolium salt. In other particular embodiments, the compound is an imidazolinium or an imidazolinium salt. In certain embodiments, the pharmaceutically acceptable salt of general formula I may be a chloride, bromide, tetrafluoroborate or hexafluorophosphate salt.

R⁵ may be the same as R⁶. R⁵ and R⁶ may be hydrocarbons. R⁷ may be H.

In various embodiments, the compound may be 1-ethyl-3-methylimidazolium, 1,3-bisbenzylimidazolium, 1,3-diisopropylimidazolium, 1,3-di-tert-butylimidazolinium, 1,3-bis(1-adamantyl)imidazolium, 1,3-bis(2,4,6-trimethylphenyl)-imidazolinium, 1,3-bis(2,6-diisopropyl-phenyl)-imidazolinium, 1,3,-bisbenzylbenzimidazolium, 1,3 dibenzyl -2-methylimidazolium, 1,3-diallylimidazolium, 1-benzyl-3-methylimidazolium, 1-butyl-3-methylimidazolium, 1-(1-adamantyl)-3-(2,4,6-trimethylphenyl)-4,5-dihydroimidazolium, 2-benzylimidazo[1,5-*a*]quinolinium, 1,3-bis(1-adamantyl)-benzimidazolium, 1,3-dicyclohexylbenzimidazolium, 1,3-diisopropylimidazolinium tetrafluoroborate, 1,3-diisopropylimidazolium, 2-(2,6-diisopropylphenyl)-5-methylimidazo[1,5-*a*]pyridinium, 1-(2,6-diisopropylphenyl)-3-(2,4,6-trimethylphenyl)-imidazolinium, 2-mesityl-5- methylimidazo[1,5-*a*]pyridinium, 2-mesityl-2,5,6,7-tetrahydropyrrolo[2,1-*c*][1,2,4]triazol-4-ium, 1,3-bis(1-adamantyl)imidazolinium, 1-butyl-3-(2-pyridinylmethyl)-1*H*-imidazolium, 6,7-dihydro-2-pentafluorophneyl-5H-pyrrolo[2,1-*c*]-1,2,4-trizolium, 1-methyl-3-(2-hydroxylethyl)-imidazolium, 1-methyl-3-(4-isocynatobenzyl)-imidazolium, 1-methyl-3-(4-carboxylbenzyl)-imidazolium, 1-methyl-3-(4-acetate-benzyl)-imidazolium, 1-methyl-3-(2,2-dimethoxylethyl)-imidazolium, 1-(2,4,6-trimethylphenyl)-3-(4-acetate-benzyl)-imidazolium, 1,3-Dibenzyl-5-phenylimidazolium, 1-benzyl-3-(4-carboxylbenzyl)-2-methylimidazolium, 1-benzyl-3-(3,4,5-trimethoxylbenzyl)-2-methylimidazolium, 1-benzyl-3-(4-acetate-benzyl)-2-methyl-imidazolium, 1-benzyl-3-(4-methylcarboxylatebenzyl)-2-methyl-imidazolium, 1-benzyl-3-(2,2-dimethoxylethyl)-2-methyl-imidazolium, 2,6-di-(3-benzyl-imidazolium)-pyridine, 2,2'-di-(3-benzyl-imidazolium)-1,1'-binaphthalene, 1-benzyl-3-(4-methylbenzyl)-2-methylimidazolium, 1-benzyl-3-(2-trifluoromethylbenzyl)-2-methylimidazolium, 1-benzyl-3-(4-methylcarboxylatebenzyl)-5-phenyl-imidazolium, 1-benzyl-3-(4-acetatebenzyl)-5-phenyl-imidazolium, 1-benzyl-3-(4-methylbenzyl)-5-phenylimidazolium, (1,2-4,5-diimidazolium)-N,N',N",N"'-tetrabenzyl-benzene, 1-benzyl-3-(2-propyn-1-yl)-imidazolium, 1-benzyl-3-(3-hydroxyl-propyl)-imidazolium, 1,3-di(2-phenylethyl)-imidazolium, 1-benzyl-3-(4-acetatebenzyl)-imidazolium, 1-benzyl-3-(pyridin-2-yl)-imidazolium, 1,3,5-tris-(4-methyl-imidazolium)-linked cyclophane, 1,3-dibenzyl-2-(1,3-dibenzyl-1H-imidazol-2(3H)-ylidene)-2,3-dihydro-1H-imidazole, 1-benzyl-3-methyl-imidazolium, 1-(4-cyanatobenzyl)-3-methyl-imidazolium, 1-(4-carboxybenzyl)-3-methyl-imidazolium, 1-methyl-3-(4-acetate-benzyl)-imidazolium, 1-methyl-3-(2,2-dimethoxyethyl)-imidazolium, or 1-(2,4,6-trimethylphenyl)-3-(4-acetate-benzyl)-imidazolium, or a pharmaceutically acceptable salt thereof.

Alternatively, the compound may be 1,3-bisbenzylimidazolium, 1,3-diisopropylimidazolium, 1,3-di-tert-butylimidazolinium, 1,3-bis(1-adamantyl)imidazolium, 1,3-bis(2,4,6-trimethylphenyl)-imidazolinium, 1,3-bis(2,6-diisopropyl-phenyl)-imidazolinium, 1,3,-bisbenzylbenzimidazolium, or 1,3,5-tris-(4-methyl-imidazolium)-linked cyclophane, or a pharmaceutically acceptable salt thereof.

In one particular embodiment, the compound is 1,3-bisbenzylimidazolium or a pharmaceutically acceptable salt thereof. In another particular embodiment, the compound is 1,3-diisopropylimidazolium or a pharmaceutically acceptable salt thereof.

The compound may be a dimer of a compound having a structure of general formula I or a pharmaceutically acceptable salt thereof. For example, the compound may be 1,3-dibenzyl-2-(1,3-dibenzyl-1H-imidazol-2(3H)-ylidene)-2,3-dihydro-1H-imidazole, (1,2-4,5-diimidazolium)-N,N',N", N"'-tetrabenzyl-benzene, 2,6-di-(3-benzyl-imidazolium)-pyridine or 2,2'-di-(3-benzyl-imidazolium)-1,1'-binaphthalene, or a pharmaceutically acceptable salt thereof.

Alternatively, the compound may be a trimer of a compound having a structure of general formula I or a pharmaceutically acceptable salt thereof. For example, the compound may be 1,3,5-tris(4-methyl-imidazolium)-linked cyclophane or a pharmaceutically acceptable salt thereof.

The neural cell may be an *in vitro* cell or may be an *in vivo* cell. For example, the method may comprise administering an effective amount of the neuroprotective agent to a subject for the treatment of a neurological disorder. Alternatively, the use may comprise use of an effective amount of the compound for treatment of a neurological disorder in a subject. The neurological disorder may be for example Parkinson's disease or retinopathy.

Other aspects and features of the present invention will become apparent to those of ordinary skill in the art upon review of the following description of specific embodiments of the invention in conjunction with the accompanying figures.

### BRIEF DESCRIPTION OF THE DRAWINGS

In the figures, which illustrate, by way of example only, embodiments of the present invention:

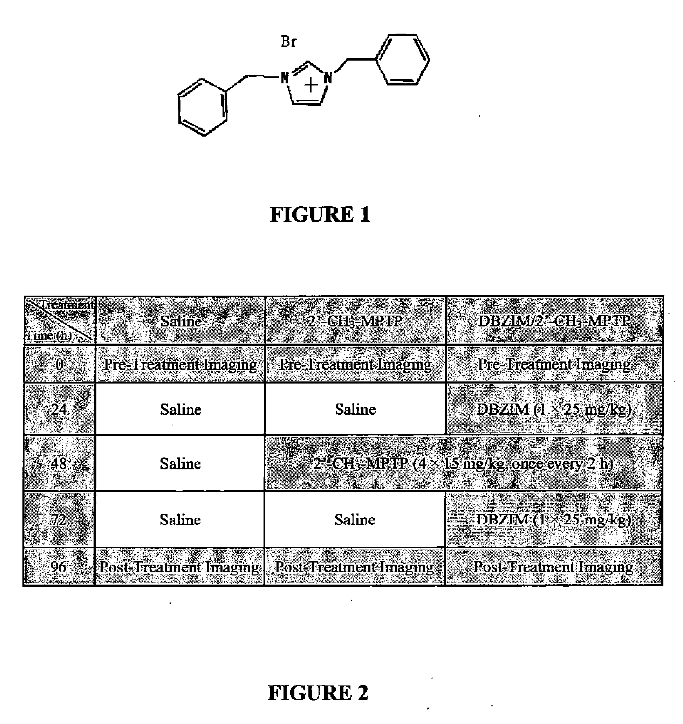

**Figure. 1****. Chemical structure of DBZIM** (1,3-bisbenzylimidazolium bromide).
**Figure. 2****. Compound dosing and retinal imaging regime.** Mice were treated with neurotoxicant 2'-CH₃-MPTP, DBZIM and neurotoxicant 2'-CH₃-MPTP or saline. The DBZIM /neurotoxicant treated group received an intraperitoneal (ip) injection of DBZIM (25 mg/kg in saline) 24 h before and after administering 4 ip injections of 2'-CH₃-MPTP (15 mg/kg in saline; once every 2 h). Mice in the neurotoxicant-treated group were injected with 2'-CH₃-MPTP only (4 × 15 mg/kg in saline; ip; once every 2 h). For each mouse in the DBZIM/neurotoxicant-treated group (*n* = 5) and neurotoxicant-treated group (*n* = 5), saline was used as a vehicle for the control group (*n* = 6).

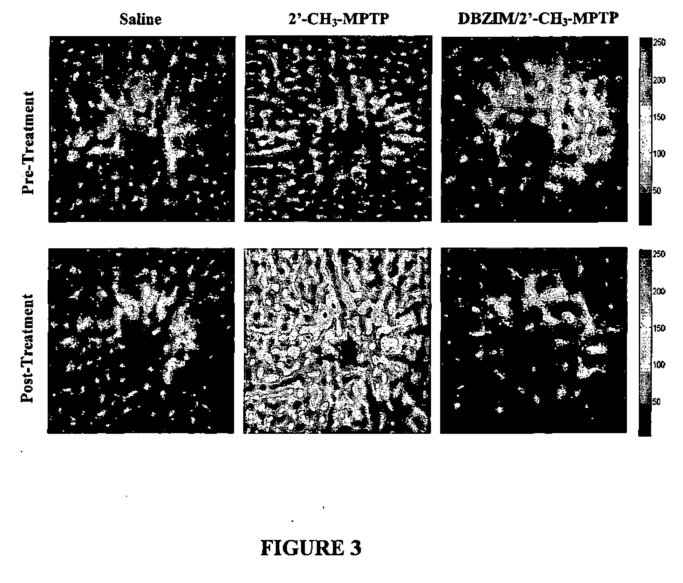

**Figure. 3****. Retinal imaging of transgenic GFAP-GFP (glial fibrillary acidic protein-green fluorescent protein) mice.** Adult mice (8-10 weeks old) in FVB/N (Friend virus B-Type) background were treated with saline, 2'-CH₃-MPTP, and DBZIM/2'-CH₃-MPTP, respectively. A basal level of GFP (green fluorescent protein) signal is seen in the optic disc. A significant elevation in the GFP signal is observed for the mouse treated with the 2'-CH₃-MPTP neurotoxicant after 96 h. It is noted that the maximal GFP fluorescence is seen around the fringe of the optic disc.

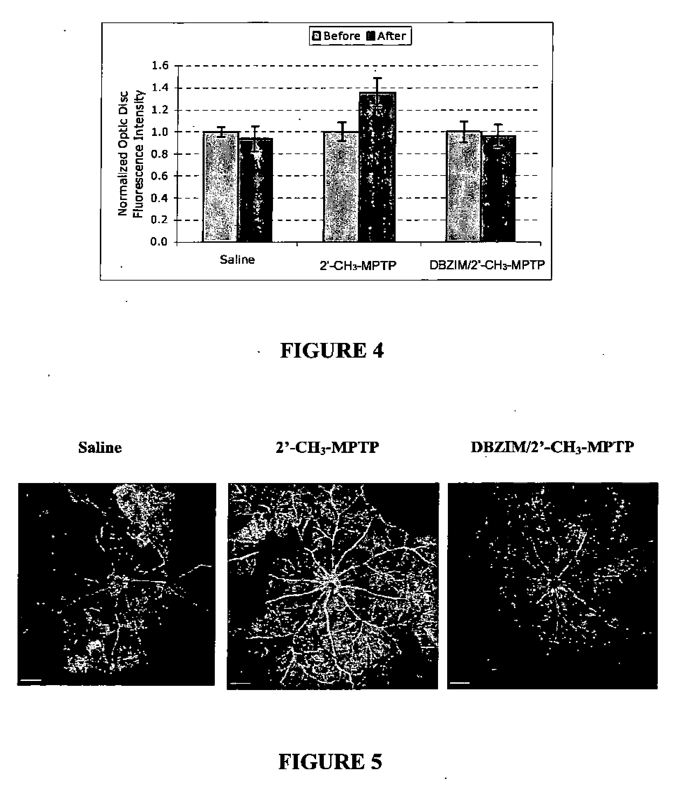

**Figure. 4****. Quantification of fluorescence intensity (FI) induced by neurotoxicant 2'-CH₃-MPTP.** Raw images were obtained from the optic discs of a total of 16 adult mice treated with saline (n = 6), 2'-CH₃-MPTP (n = 5), or DBZIM/2'-CH₃-MPTP (n = 5). After the raw images were processed, the FI (Fluorescence Intensity) values were obtained, and analyzed using the student's *t*-test. The data were expressed as mean ± SEM. 2'-CH₃-MPTP induces a significant increase (*P < 0.05) in FI over the saline control in the optic discs of the 2'-CH₃-MPTP-treated mice, signifying retinal toxicity. In contrast, DBZIM treatment prevented the 2'-CH₃-MPTP-induced retinal toxicity (measured at day 4).
**Figure. 5****. Retinal whole-mounts from mice that received three different treatments.** Left: In the saline-treated mouse, retinal astrocytes at NFL (Nerve Fiber Layer) ensheathing blood vessels radiating from the center of the optic disc are clearly visible with the innate GFP fluorescence (green) and the GFAP immunochemistry (red). In addition, the end-feet of Müller cells appear as punctated dots in the vessel-free area smaller than the astrocytes. Middle: The neurotoxicant induces gliosis as evidenced by an increase in the total green and red staining in both astrocytes and Müller cells. The number of glial cells as marked by GFP seems to increase as well (hyperplasia). Right: DBZIM significantly attenuates the gliosis, as shown by a decrease in both green and red staining. Bar = 200 µm.

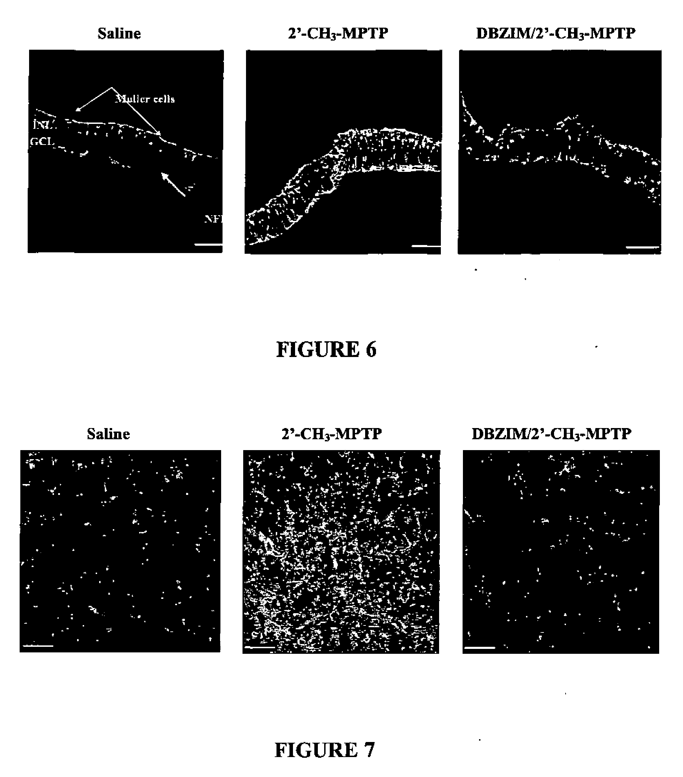

**Figure. 6****. DBZIM attenuates 2'-CH₃-MPTP-induced gliosis in retinal astrocytes and Müller cells**. Left: Astrocytes in the saline control retina at the NFL (Nerve Fiber Layer) and GCL (Ganglion Cell Layer) are labeled by the innate GFP fluorescence (green) and the GFAP antibody (red), whereas Müller cells in the INL (Inner Nuclear Layer) are predominantly labeled by the transgenic GFP. Middle: The neurotoxicant dramatically intensifies the GFP and GFAP staining in both astrocytes and Müller cells, indicating severe retinal toxicity and gliosis. Right: With the DBZIM treatment, the **2'-CH₃-MPTP-induced** gliosis is significantly attenuated in both astrocytes and Müller cells. Bar = 50 µm.
**Figure. 7****. DBZIM attenuates 2'-CH₃-MPTP-induced gliosis.** Left: The striatal astrocytes in the saline control group express a basal level of GFP, indicating a lack of toxicity. The DA (dopaminergic) axons are labeled by the TH (tyrosine hydroxylase) antibody as red dots. Middle: The neurotoxicant dramatically induces gliosis in this area as evidenced by an elevation in GFP signal. Due to the low basal level of TH in the axons, no significant change in the TH expression is visually observed. Right: DBZIM significantly attenuates the neurotoxicity in this area. Bar = 50 µm.

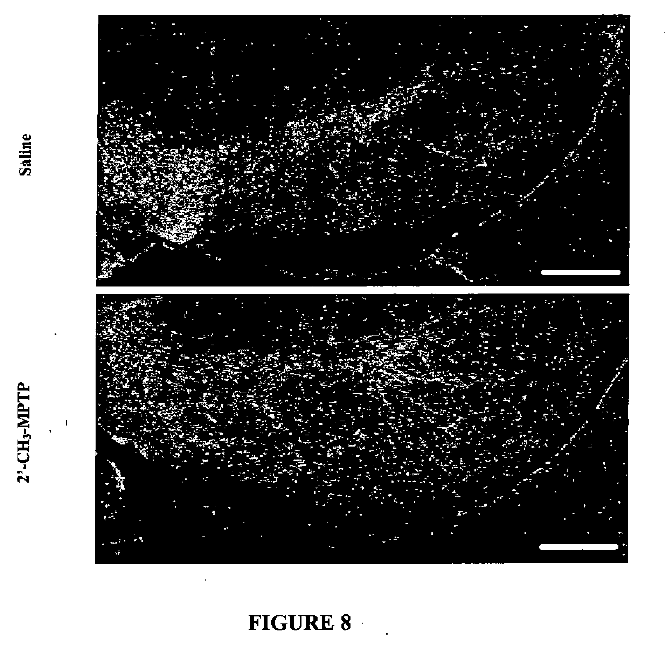

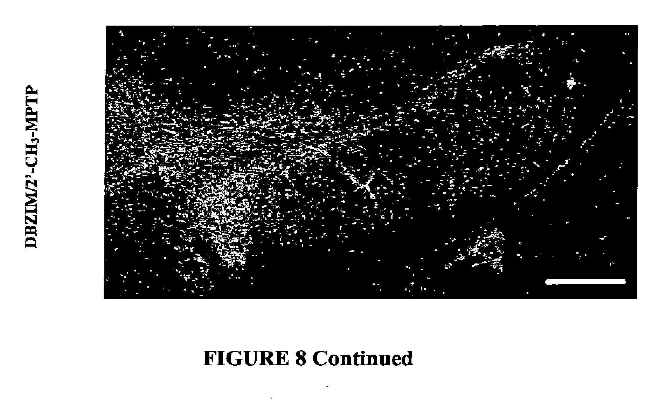

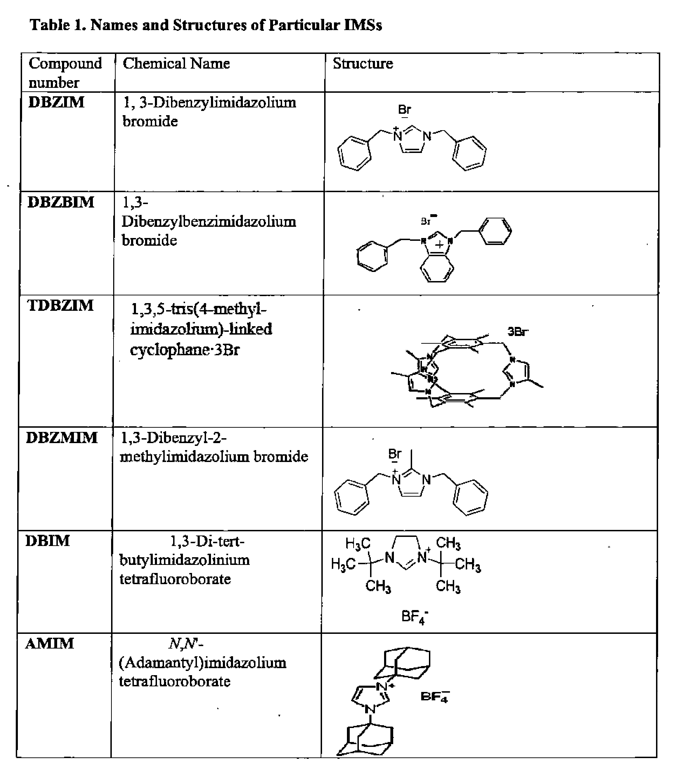

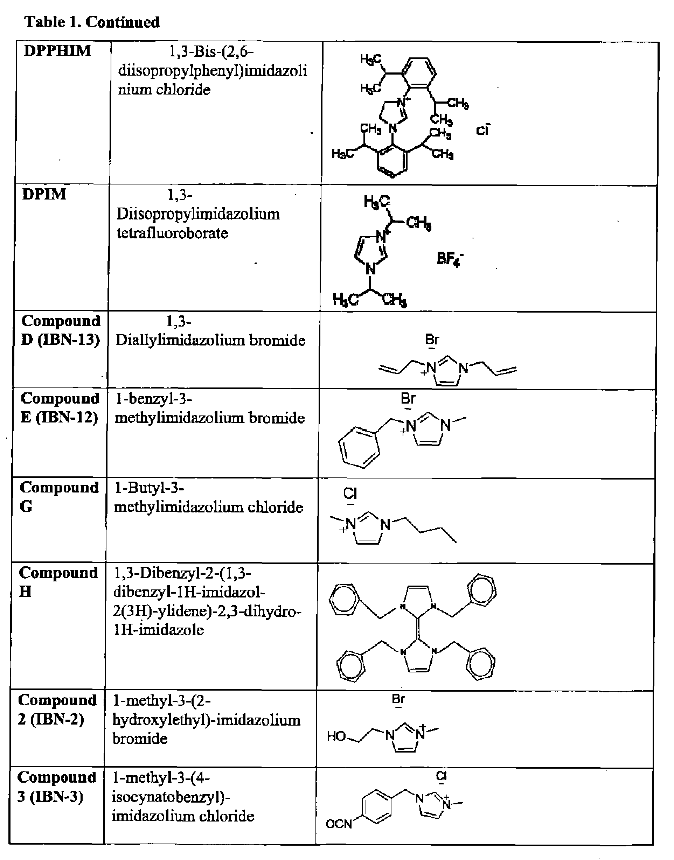

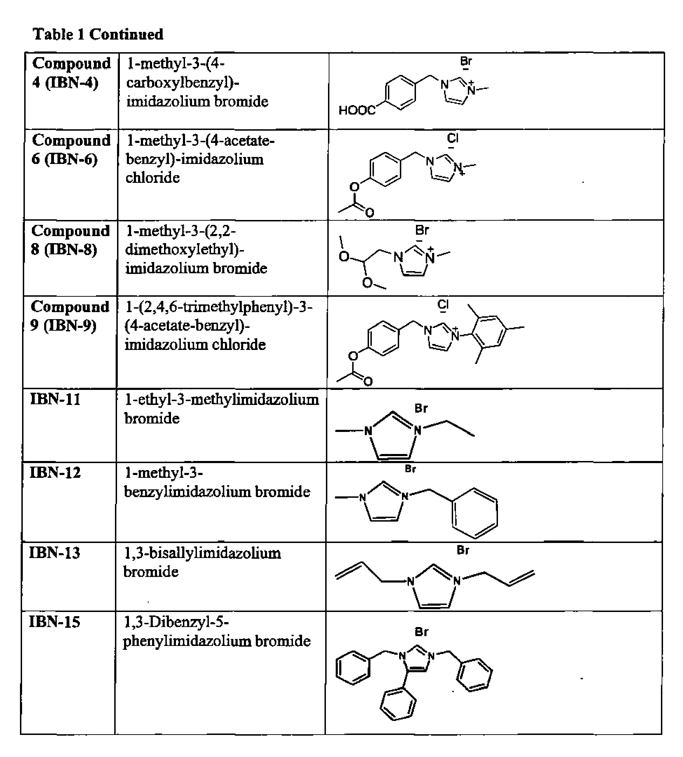

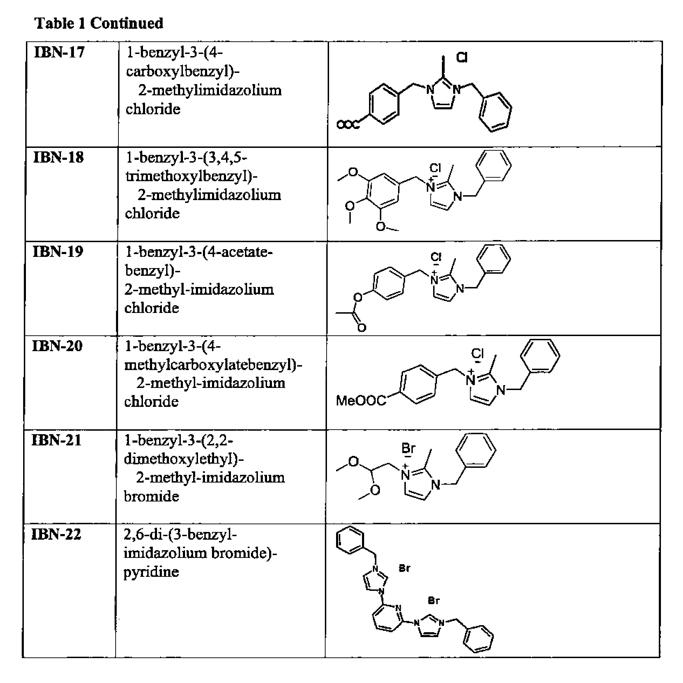

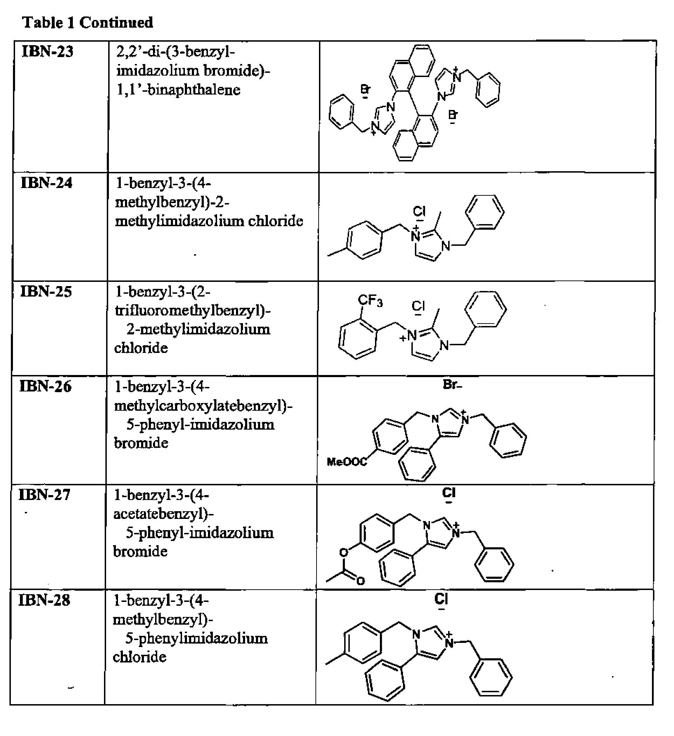

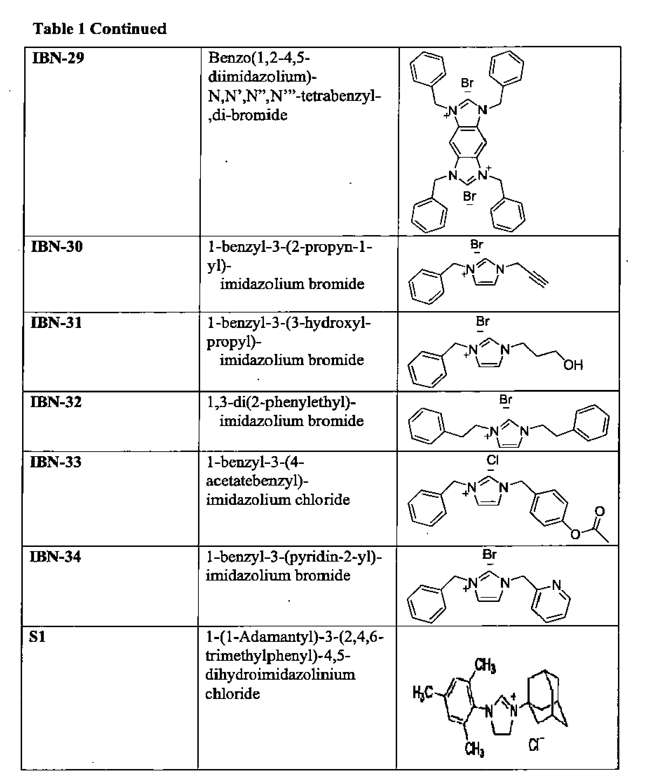

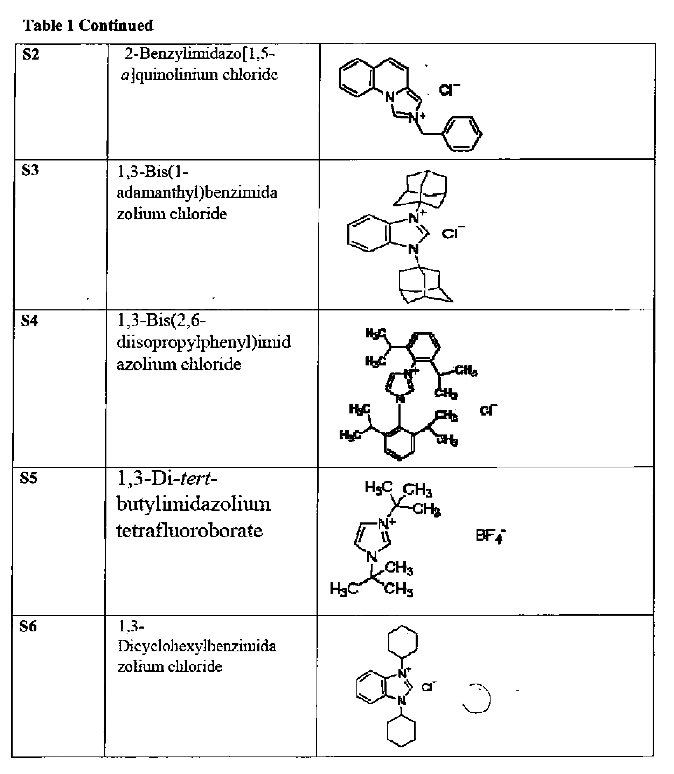

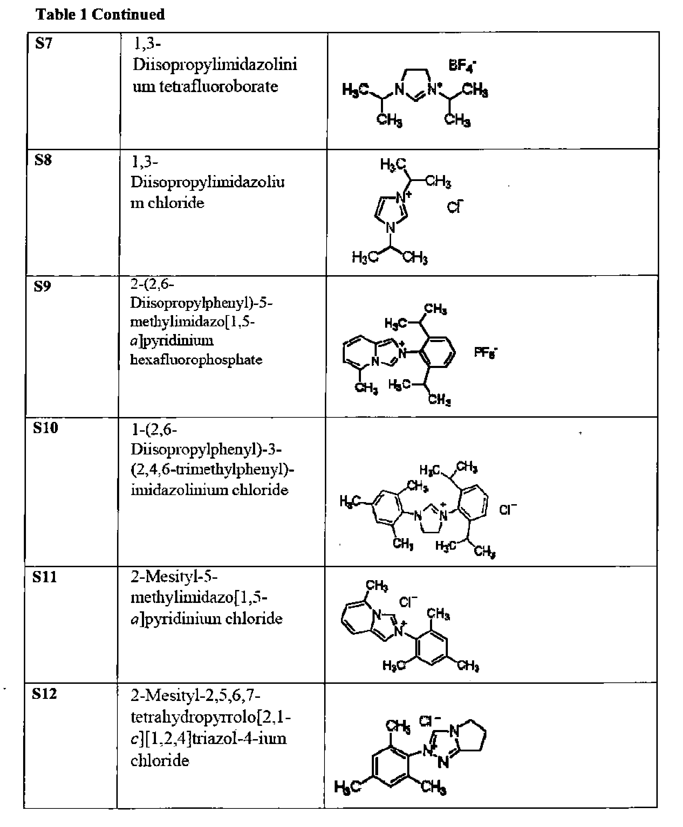

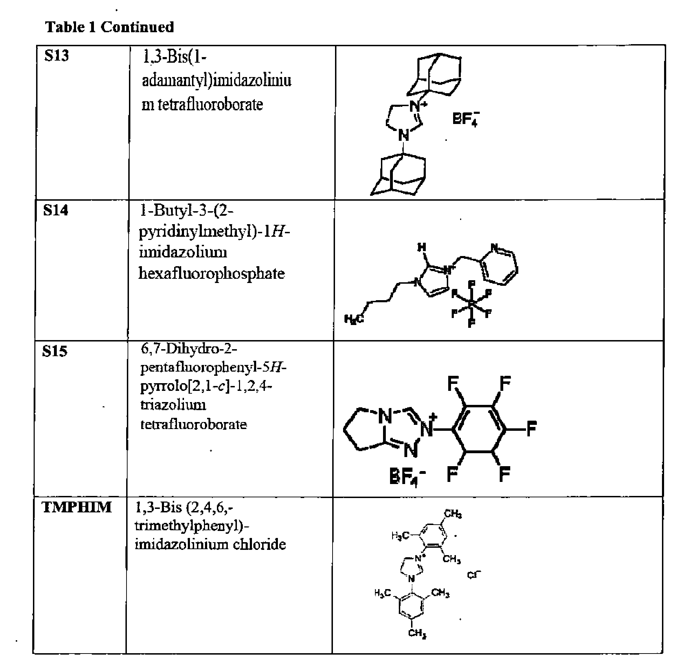

**Figure. 8****. DBZIM attenuates 2'-CH₃-MPTP-induced DA neuron depletion.** Top: The normal-sized DA neuron cluster is stained with antibody against TH (in red), and the resting astrocytes are moderately labeled by the GFP (green). Middle: A significant number of DA neurons are acutely ablated by the neurotoxicant from the SNpc (substantia nigra pars compacta), accompanied by an increase in GFP signal in the surrounding astrocytes. Bottom: A significant portion of the DA neurons is protected with DBZIM treatment, as evidenced by the TH staining (red). The persistence in GFP elevation (gliosis) in the vicinity of SNpc indicates that some 2'-CH₃-MPTP-induced neurotoxicity still exists. Bar = 200 µm.

**Table 1. Names and Structures of IMSs.**

### DETAILED DESCRIPTION

The methods and uses described herein relate to the discovery that certain imidazolium and imidazolinium compounds (collectively "IMSs") as described in general formula I below, including in the form of imidazolium and imidazolinium salts for example as shown in general formula II below, may be used as neuroprotective agents or to treat neurological disorders.

Both imidazoliums and imidazoliniums are based on an imidazole ring and both are N,N'-substituted; imidazoliums are N,N'-substituted imidazoles, while imidazoliniums are N,N'-substituted imidazolines and do not have the carbon-carbon double bond between positions C4 and C5 that is present in imidazole. Imidazole itself is incorporated in many biological molecules, and synthetic C-substituted imidazoles have become an important part of many pharmaceuticals (Olmos et al. 1999) (Casanovas et al. 2000).

One attractive feature of using IMSs in synthetic chemistry is the structural versatility they provide. The electronic structure and stability, and thus the therapeutic safety and efficacy of IMSs, can be fine-tuned by varying the N-substituents (substituents on the nitrogen atoms of the central ring) and the central ring of the molecule. It will be understood that the "central ring" of IMSs refers to the five membered ring containing at least 2 nitrogen atoms to which various substituents may be bound to create different IMS molecules and thus refers to either the imidazole ring or imidazoline ring. IMSs provide inexpensive and chemically tunable building blocks for the development of novel therapeutics.

Some IMSs are precursors of N-heterocyclic carbenes (NHCs). NHCs can be easily generated from IMSs having a hydrogen atom at the C2 position of the central ring and substituents on both nitrogen atoms of the central ring i.e. two N-substituents. NHCs are generated from these IMSs by the deprotonation of the IMS under the appropriate conditions. Deprotonation of IMSs may be carried out under basic conditions or in a diluted solution under neutral conditions. It may be difficult to generate NHCs from IMSs with substituents other than a hydrogen atom at the C2 position of the central ring. However, radical species may be formed from these IMSs by cleaving one or both of the N-substituents.

The inventors have discovered that the IMSs as described herein have neuroprotective properties. Neurological disorders have been linked to oxidative stress response and, without being limited to any particular theory, the IMSs as described herein may exhibit anti-oxidative properties.

IMSs that may be used as neuroprotective agents are compounds having a structure of the general formula I:

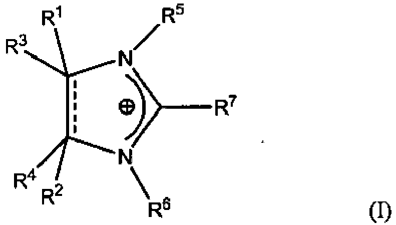

In general formula I, the dashed line is absent or is present as a bond to form a second bond between the carbon to which R¹ and R³ are attached and the carbon to which R² and R⁴ are attached. Thus, the carbon to which R¹ and R³ are attached and the carbon to which R² and R⁴ are attached may be connected by either a single bond or a double bond.

R¹ and R² (i) are each independently H, straight or branched C₁-C₆ alkyl, straight or branched C₂-C₆ alkenyl, straight or branched C₂-C₆ alkynyl, C₆-C₁₀ aryl; or (ii) together with their central ring atoms form a 6- to 10-membered fused saturated, unsaturated or aromatic ring system; or (iii) R¹ and R⁵ together with their central ring atoms or R² and R⁶ together with their central ring atoms, form a 5- to 10-membered fused saturated, unsaturated or aromatic ring system and the other of R¹ and R² is as defined above in (i); or (iv) R¹ and R⁵ together with their central ring atoms and R² and R⁶ together with their central ring atoms each independently form a 5- to 10-membered fused saturated, unsaturated or aromatic ring system.

R³ and R⁴ are both H, or, when R¹ and R² together with their central ring atoms form a 6- to 10-membered fused aromatic ring system or when the dashed line is present as a bond, R³ and R⁴ are absent.

When R⁵ or R⁶ do not form a fused aromatic ring together with R¹ or R², respectively, as set out above, R¹ and R⁶ are each independently straight or branched C₁-C₆ alkyl, straight or branched C₂-C₆ alkenyl, straight or branched C₂-C₆ alkynyl, C₃-C₁₈ cycloalkyl including fused cycloalkyl ring systems, C₆-C₁₀ aryl, C₆-C₁₀ aryl-C₁-C₆ alkyl, C₆-C₁₀ aryl-C₂-C₆ alkenyl, or C₆-C₁₀ aryl-C₂-C₆ alkynyl, C₁-C₆ alkyl-C₆-C₁₀ aryl, C₂-C₆ alkenyl-C₆-C₁₀ aryl, or C₂-C₆ alkynyl-C₆-C₁₀ aryl.

R⁷ may be H, C₁-C₆ alkyl or C₆-C₁₀ aryl.

Any of the above substituents R¹ to R⁶, where applicable, may optionally have one or more carbon atoms replaced with a heteroatom selected from N, O, S and P. As well, any of the above substituents R¹ to R⁶, where applicable, may optionally be substituted with one or more of straight or branched C₁-C₆ alkyl, straight or branched C₂-C₆ alkenyl, straight or branched C₂-C₆ alkynyl, C₃-C₁₈ cycloalkyl including fused cycloalkyl ring systems, C₆-C₁₀ aryl, fluoro, tri-fluoro-methyl, cyanato, isocyanato, carboxyl, C₁-C₆ acyloxy, C₁-C₆ acyl, carbonyl, amino, acetyl, acetoxy, oxo, nitro, hydroxyl, C₁-C₆ alkylcarboxy, C₁-C₆ alkoxy, C₂-C₆ alkenoxy, C₂-C₆ alkynoxy.

One of the central ring carbon atom to which R¹ and R³ are attached and the central ring carbon atom to which R² and R⁴ are attached may be optionally replaced with a nitrogen atom.

The above compounds may be present in salt form. Thus, IMSs that may be used in the present methods include pharmaceutically acceptable salts of compounds of general formula I and oligomers and polymers of such salts. Such salts may have a structure of the general formula II:

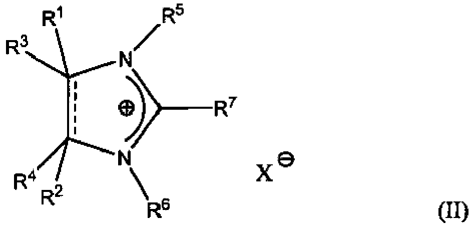

General formula II is as defined above for general formula I, with the further feature of counterion X. X is a pharmaceutically acceptable anion, including but not limited to chloride, bromide, tetrafluoroborate, hexafluorophosphate.

The term "fused" as used herein in reference to ring structures refers to the sharing of at least two atoms between ring structures. When two central ring atoms (either of which may be C or N) are included in a fused ring system, the ring system is fused to the central imidazolium or imidazolinium ring.

In particular embodiments, the IMS has a structure of general formula I or II, in which the dashed line may be absent or is present as a bond to form a second bond between the carbon to which R¹ and R³ are attached and the carbon to which R² and R⁴ are attached; R¹ and R² are both H or together with their central ring atoms form a 6- to 10-membered fused aromatic ring system; R³ and R⁴ are both H or are absent; R⁵ and R⁶ are each independently straight or branched C₁-C₆ alkyl, straight or branched C₂-C₆ alkenyl, straight or branched C₂-C₆ alkenyl, C₃-C₁₈ cycloalkyl including fused cycloalkyl ring systems, C₆-C₁₀ aryl, C₆-C₁₀ aryl-C₁-C₆ alkyl, C₆-C₁₀ aryl-C₂-C₆ alkenyl or C₆-C₁₀ aryl-C₂-C₆ alkynyl; R⁷ is H, methyl or phenyl; any of which substituents R¹ to R⁶, where applicable may have one or more carbon atoms replaced with a heteroatom selected from N, O, S and P and may optionally be substituted with one or more of straight or branched C₁-C₆ alkyl, straight or branched C₂-C₆ alkenyl, straight or branched C₂-C₆ alkynyl, C₃-C₁₈ cycloalkyl including fused cycloalkyl ring systems, C₆-C₁₀ aryl, fluoro, tri-fluoro-methyl, cyanato, carboxyl, carbonyl, amino, acetyl, oxo, nitro, C₁-C₆ alkoxy, C₂-C₆ alkenoxy, C₂-C₆ alkynoxy.

Alternatively, the IMS has the structure of general formula I or general formula II, in which the dashed line is absent or is present as a bond to form a second bond between the carbon to which R¹ and R³ are attached and the carbon to which R² and R⁴ are attached. Alternatively, the IMS has the structure of general formula I or general formula II, in which R¹ and R² are both H or together with their central ring atoms form a 6- to 10-membered fused aromatic ring system. Alternatively, the IMS has the structure of general formula I or general formula II, in which R³ and R⁴ are both H or are absent. Alternatively, the IMS has the structure of general formula I or general formula II, in which R⁵ and R⁶ are each independently straight or branched C₁-C₆ alkyl, straight or branched C₂-C₆ alkenyl, straight or branched C₂-C₆ alkynyl, C₃-C₁₈ cycloalkyl including fused cycloalkyl ring systems, C₆-C₁₀ aryl, C₆-C₁₀ aryl-C₁-C₆ alkyl, C₆-C₁₀ aryl-C₂-C₆ alkenyl or C₆-C₁₀ aryl-C₂-C₆ alkynyl. Alternatively, the IMS has the structure of general formula I or general formula II, in which R⁷ is H, methyl or phenyl. Alternatively, the IMS has the structure of general formula I or general formula II, in which any of R¹ to R⁶, where applicable has one or more carbon atoms replaced with a heteroatom selected from N, O, S and P. Alternatively, the IMS has the structure of general formula I or general formula II, in which any of R¹ to R⁶, where applicable is substituted with one or more of straight or branched C₁-C₆ alkyl, straight or branched C₂-C₆ alkenyl, straight or branched C₂-C₆ alkynyl, C₃-C₁₈ cycloalkyl including fused cycloalkyl ring systems, C₆-C₁₀ aryl, fluoro, tri-fluoromethyl, cyanato, carboxyl, carbonyl, amino, acetyl, oxo, nitro, C₁-C₆ alkoxy, C₂-C₆ alkenoxy, C₂-C₆ alkynoxy.

For example, in various embodiments, the compound may be an imidazolium salt, with a double bond between the two central ring atoms to which R¹ and R² are attached. In various other embodiments, the compound may be an imidazolinium salt, wherein R³ and R⁴ are hydrogens and there is only a single bond between their central ring carbons. In yet other embodiments, R³ and R⁴ are absent and R¹ and R² together with their central ring atoms form a 6- to 10-membered fused aromatic ring system.

The substituents comprising R⁵ and R⁶ may also vary. For example, in some embodiments, the IMS of the present method may be a compound wherein R⁵ and R⁶ are the same. In different embodiments, R⁵ or R⁶ may be aralkyls, branched alkyls or cycloalkyls including fused ring systems. In other embodiments R⁵ or R⁶ may comprise a phenalkyl group. In other embodiments, one or both of R⁵ and R⁶ may comprise an adamantanyl group.

IMSs that may be used in the present method also include oligomers or polymers formed from compounds of general formula I or general formula II. Thus, "oligomer" as used herein, refers to, for example, 2 or more, 3 or more, 4 or more, 5 or more, 10 or more, 15 or more, 20 or more, 25 or more, 50 or more, 100 or more, 150 or more compounds of general formula I or general formula II connected together in the manner described below. Thus, oligomer includes dimers and trimers. "Polymers" as used herein refers to, for example, 100 or more, 150 or more, 200 or more, 250 or more, 500 or more, 1000 or more compounds of general formula I or general formula II connected together in the manner described below.

In oligomers or polymers as used herein, compounds of general formula I or general formula II are connected to each other to form a macromolecular structure, which may be cyclised.

In the macromolecular structure, two compounds of general formula I or general formula II may be connected together so that one or more R¹, R², R⁵, R⁶ or R⁷ substituent is bivalent and is thus shared between the two compounds, rather than have a relevant substituent present in the oligomer or polymer for every compound of general formula I or general formula II present. For example, in such a dimer, two compounds may be connected by a shared bivalent R¹ substituent, and thus in the complete dimer, there is only one occurrence of an R¹ substituent for the two compounds of general formula I or general formula II.

Thus, the compounds of general formula I or general formula II may be joined to each other via a bivalent substituent such as R¹, R², R⁵, R⁶, or R⁷. For example, an R⁵ substituent on one compound of general formula I may also be R⁵ on a second compound of general formula I such that the two compounds are linked via a shared R⁵ substituent. Similarly, oligmers or polymers may be formed by linking compounds via shared R¹, R², R⁵, R⁶, or R⁷ substituents.

For example, dimers of this description include 2,6-di-(3-benzyl-imidazolium bromide)-pyridine or a pharmaceutically acceptable salt thereof (IBN-22).

For example, trimers as described herein include 1,3,5-tris(4-methyl-imidazolium)-linked cyclophane or a pharmaceutically acceptable salt thereof (TDBZIM).

Sharing of substituents as described above can include sharing of two substituents that together with their central ring carbons form a fused ring system. Thus, for example, if R¹ and R² together with their central ring carbons form a ring system on a first compound of general formula I, additional carbons within the ring will be central ring carbons from a second compound of general formula I. For example, dimers of this description include benzo(1,2-4,5-diimidazolium)-N,N',N", N"'-tetrabenzyl-di-bromide or a pharmaceutically acceptable salt thereof (IBN-29).

Alternatively, a substituent such as one or more of R¹, R², R⁵, R⁶ or R⁷ may be bonded to another such substituent on a second compound in order to connect two compounds together to form an oligomer or polymer, and thus there will be one occurrence of the relevant linking substituent for each compound of general formula I in the oligomer or polymer. Multiple compounds falling within general formula I or general formula II may be joined together (oligomerised) in this way, by one or more shared bivalent R¹, R², R⁵, R⁶ or R⁷ substituents. For example, such dimers include 2,2'-di-(3-benzyl-imidazolium bromide)-1,1'-binaphthalene or a pharmaceutically acceptable salt thereof (IBN-23).

Additionally, two compounds of general formula I or general formula II may be attached by a single or double bond between respective carbon atoms to which any of R¹, R² or R⁷ is attached in general formula I or general formula II to form an oligomer or polymer, including a dimer. It will be appreciated that when two compounds are attached via a single or double bond to form such an oligomer or polymer, then the relevant atom of the central ring will have the appropriate substituents (including hydrogen atoms) replaced by this single or double bond. These molecules include for example, 1,3-dibenzyl-2-(1,3-dibenzyl-1H-imidazal-2(3H)-ylidene)-2,3-dihydro-1H-imidazole or a pharmaceutically acceptable salt thereof (compound H).

The oligomerised or polymerised compounds may be the same compound, such that all substituents in general formula I or general formula II are the same in each oligomerised or polymerised compound, or may be different, with one or more substituents differing between compounds, with the exception that shared bivalent substituent or substituents will obviously be the same in the compounds in which the bivalent substituent is shared.

Table 1 contains the names and structures of particular examples of the IMSs described herein.

In particular embodiments, the compound of the present invention may be 1-ethyl-3-methylimidazolium, 1,3-bisbenzylimidazolium, 1,3-diisopropylimidazolium, 1,3-di-tert-butylimidazolinium, 1,3-bis(1-adamantyl)imidazolium, 1,3-bis(2,4,6-trimethylphenyl)-imidazolinium, 1,3-bis(2,6-diisopropyl-phenyl)-imidazolinium, 1,3,-bisbenzylbenzimidazolium, 1,3-dibenzyl -2 methylimidazolium, 1,3-diallylimidazolium, 1-benzyl-3-methylimidazolium, 1-butyl-3-methylimidazolium, 1-(1-adamantyl)-3-(2,4,6-trimethylphenyl)-4,5-dihydroimidazolium, 2-benzylimidazo[1,5-α]quinolinium, 1,3-bis(1-adamantyl)-benzimidazolium, 1,3-dicyclohexylbenzimidazolium, 1,3-diisopropylimidazolinium, 1,3-diisopropylimidazolium, 2-(2,6-diisopropylphenyl)-5-methylimidazo[1,5-*a*]pyridinium, 1-[2,6-diisopropylphenyl)-3-(2,4,6-trimethylphenyl)-imidazolinium, 2-mesityl-5- methylimidazo[1,5-*a*]pyridinium, 2-mesityl-2,5,6,7-tetrahydropyrrolo[2,1-*c*][1,2,4]triazol-4-ium, 1,3-bis(1-adamantyl)imidazolinium, 1-butyl-3-(2-pyridinylmethyl)-1*H*-imidazolium, 6,7-dihydro-2-pentafluorophneyl-5H-pyrrolo[2,1-*c*]-1,2,4-trizolium, 1-methyl-3-(2-hydroxylethyl)-imidazolium, 1-methyl-3-(4-isocynatobenzyl)-imidazolium, 1-methyl-3-(4-carboxylbenzyl)-imidazolium, 1-methyl-3-(4-acetate-benzyl)-imidazolium, 1-methyl-3-(2,2-dimethoxylethyl)-imidazolium, 1-(2,4,6-trimethylphenyl)-3-(4-acetate-benzyl)-imidazolium, 1,3-Dibenzyl-5-phenylimidazolium, 1-benzyl-3-(4-carboxylbenzyl)-2-methylimidazolium, 1-benzyl-3-(3,4,5-trimethoxylbenzy)-2-methylimidazolium, 1-benzyl-3-(4-acetate-benzyl)-2-methyl-imidazolium, 1-benzyl-3-(4-methylcarboxylatebenzyl)-2-methyl-imidazolium, 1-benzyl-3-(2,2-dimethoxylethyl)-2-methyl-imidazolium, 2,6-di-(3-benzyl-imidazolium)-pyridine, 2,2'-di-(3-benzyl-imidazolium)-1,1'-binaphthalene, 1-benzyl-3-(4-methylbenzyl)-2-methylimidazolium, 1-benzyl-3-(2-trifluoromethylbenzyl)-2-methylimidazolium, 1-benzyl-3-(4-methylcarboxylatebenzyl)-5-phenyl-imidazolium, 1-benzyl-3-[4-acetatebenzyl)-5-phenyl-imidazolium, 1-benzyl-3-(4-methylbenzyl)-5-phenylimidazolium, (1,2-4,5-diimidazolium)-N,N',N",N"'-tetrabenzyl-benzene, 1-benzyl-3-(2-propyn-1-yl)-imidazolium, 1-benzyl-3-(3-hydroxyl-propyl)-imidazolium, 1,3-di(2-phenylethyl)-imidazolium, 1-benzyl-3-(4-acetatebenzyl)-imidazolium, 1-benzyl-3-(pyridin-2-yl)-imidazolium, 1,3,5-tris-(4-methyl-imidazolium)-linked cyclophane, 1,3-dibenzyl-2-(1,3-dibenzyl-1H-imidazol-2(3H)-ylidene)-2,3-dihydro-1H-imidazole, 1-benzyl-3-methyl-imidazolium, 1-(4-cyanatobenzyl)-3-methyl-imidazolium, 1-(4-carboxybenzyl)-3-methyl-imidazolium, 1-methyl-3-(4-acetate-benzyl)-imidazolium, 1-methyl-3-(2,2-dimethoxyethyl)-imidazolium, or 1-(2,4,6-trimethylphenyl)-3-(4-acetate-benzyl)-imidazolium, or any pharmaceutically acceptable salt thereof.

In particular embodiments, the compound of the present invention may be 1-ethyl-3-methylimidazolium bromide, 1,3-bisbenzylimidazolium bromide (TDBZIM), 1,3-diisopropylimidazolium tetrafluoroborate (DPIM), 1,3-di-tert-butylimidazoliniumtetrafluoroborate (DBIM), 1,3-bis(1-adamantyl)imidazolium tetrafluoroborate (AMIM), 1,3-bis(2,4,6-trimethylphenyl)-imidazolinium chloride (TMPHIM), 1,3-bis(2,6-diisopropyl-phenyl)-imidazolinium chloride (DPPHIM), 1,3,-bisbenzylbenzimidazolium bromide (DBZBIM), 1,3-dibenzyl -2-methylimidazolium bromide (DBZMIM), 1,3-diallylimidazolium bromide (Compound D), 1-benzyl-3-methylimidazolium bromide (Compound E), 1-butyl-3-methylimidazolium chloride (Compound G), 1-(1-adamantyl)-3-(2,4,6-trimethylphenyl)-4,5-dihydroimidazolium chloride (Compound S1), 2-benzylimidazo[1,5-*a*]quinolinium chloride (Compound S2), 1,3-bis(1-adamantyl)-benzimidazolium chloride (Compound S3), 1,3-dicyclohexylbenzimidazolium chloride (Compound S6), 1,3-diisopropylimidazolinium tetrafluoroborate (Compound S7), 1,3-diisopropylimidazolium chloride (Compound **S8**), 2-(2,6-diisopropylpheneyl)-5-methylimidazo[1,5-*a*]pyridinium hexafluorophosphate (Compound S9), 1-(2,6-diisopropylphenyl)-3-(2,4,6-trimethylphenyl)-imidazolinium chloride (Compound S10), 2-mesityl-5- methylimidazo[1,5-*a*]pyridinium chloride (Compound S11), 2-mesityl-2,5,6,7-tetrahydropyrrolo[2,1-*c*][1,2,4]triazol-4-ium chloride (Compound S12), 1,3-bis(1-adamantyl)imidazolinium tetrafluoroborate (Compound S13), 1-butyl-3-(2-pyridinylmethyl)-1*H*-imidazolium hexafluorophosphate (Compound S14), 6,7-dihydro-2-pentafluorophneyl-5H-pyrrolo[2,1-*c*]-1,2,4-trizolium tetrafluroborate

(Compound S15), 1-methyl-3-(2-hydroxylethyl)-imidazolium bromide (IBN-2), 1-methyl-3-(4-isocynatobenzyl)-imidazolium chloride (IBN-3), 1-methyl-3-(4-carboxylbenzyl)-imidazolium bromide (IBN-4), 1-methyl-3-(4-acetate-benzyl)-imidazolium chloride (IBN-6), 1-methyl-3-(2,2-dimethoxylethyl)-imidazolium bromide (IBN-8), 1-(2,4,6-trimethylphenyl)-3-(4-acetate-benzyl)-imidazolium chloride (IBN-9), 1,3-Dibenzyl-5-phenylimidazolium bromide (IBN-15), 1-benzyl-3-(4-carboxylbenzyl)-2-methylimidazolium chloride (IBN-17), 1-benzyl-3-(3,4,5-trimethoxylbenzyl)-2-methylimidazolium chloride (IBN-18), 1-benzyl-3-(4-acetate-benzyl)-2-methyl-imidazolium chloride (IBN-19), 1-benzyl-3-(4-methylcarboxylatebenzyl)-2-methyl-imidazolium chloride (IBN-20), 1-benzyl-3-(2,2-dimethoxylethyl)-2-methyl-imidazolium bromide (IBN-21), 2,6-di-(3-benzyl-imidazolium bromide)-pyridine (IBN-22), 2,2'-di-(3-benzyl-imidazolium bromide)-1,1'-binaphthalene (IBN-23), 1-benzyl-3-(4-methylbenzyl)-2-methylimidazolium chloride (IBN-24), 1-benzyl-3-(2-trifluoromethylbenzyl)-2-methylimidazolium chloride (IBN-25), 1-benzyl-3-(4-methylcarboxylatebenzyl)-5-phenyl-imidazolium bromide (IBN-26), 1-benzyl-3-(4-acetatebenzyl)-5-phenyl-imidazolium bromide [IBN-27), 1-benzyl-3-(4-methylbenzyl)-5-phenylimidazolium chloride (IBN-28), Benzo(1,2-4,5-diimidazolium)-N,N'N",N'"-tetrabenzyl-,di-bromide (IBN-29), 1-benzyl-3-(2-propyn-1-yl)-imidazolium bromide (IBN-30), 1-benzyl-3-(3-hydroxyl-propyl)-imidazolium bromide (IBN-31), 1,3-di(2-phenylethyl)-imidazolium bromide (IBN-32), 1-benzyl-3-(4-acetatebenzyl)-imidazolium chloride (IBN-33), 1-benzyl-3-(pyridin-2-yl)-imidazolium bromide (IBN-34), 1,3,5-tris-(4-methyl-imidazolium)-linked cyclophane 3Br (TDBZIM), 1,3-dibenzyl-2-(1,3-dibenzyl-1H-imidazol-2(3H)-ylidene)-2,3-dihydro-1H-imidazole (compound H), 1-benzyl-3-methyl-imidazolium bromide (compound 12), 1-(4-cyanatobenzyl)-3-methyl-imidazolium chloride, 1-(4-carboxybenzyl)-3-methyl-imidazolium bromide, 1-methyl-3-(4-acetate-benzyl)-imidazolium chloride, 1-methyl-3-(2,2-dimethoxyethyl)-imidazolium bromide, or 1-(2,4,6-trimethylphenyl)-3-(4-acetate-benzyl)-imidazolium chloride.

In other particular embodiments, the compound of the present invention may be 1,3-bisbenzylimidazolium, 1,3-diisopropylimidazolium, 1,3-di-tert-butylimidazolinium, 1,3-bis(1-adamantyl)imidazolium, 1,3-bis(2,4,6-trimethylphenyl)-imidazolinium, 1,3-bis(2,6-diisopropyl-phenyl)-imidazolinium, 1,3,-bisbenzylbenzimidazolium, or 1,3,5-tris-(4-methyl-imiazolium)-linked cyclophane, or any pharmaceutically acceptable salt thereof including for example 1,3-bisbenzylimidazolium bromide (TDBZIM), 1,3-diisopropylimidazolium tetrafluoroborate (DPIM], 1,3-di-tert-butylimidazoliniumtetrafluoroborate (DBIM), 1,3-bis(1-adamantyl)imidazolium tetrafluoroborate (AMIM), 1,3-bis(2,4,6-trimethylphenyl)-imidazolinium chloride (TMPHIM), 1,3-bis(2,6-diisopropylphenyl)-imidazolinium chloride (DPPHIM), 1,3,-bisbenzylbenzimidazolium bromide (DBZBIM) or 1,3,5-tris-(4-methyl-imiazolium)-linked cyclophane-3Br (TDBZIM).

In one embodiment, the compound of the present invention may be 1,3-bisbenzylimidazolium or a pharmaceutically acceptable salt thereof, including for example 1,3-bisbenzylimidazolium bromide (DBZIM).

In another embodiment, the compound of the present invention may be 1,3-diisopropylimidazolium or a pharmaceutically acceptable salt thereof, including for example 1,3-diisopropylimidazolium tetrafluoroborate (DPIM).

The compounds of general formula I and compounds of general formula II and oligomers and polymers thereof are commercially available or may be synthesized using routine chemical methods, including as described in the Examples set out below. Methods of synthesis have also been described in Harlow et al. 1996, Zhang et al., Organic Letters, 2007; Chianese and Cratree 2005 and Boydston et al. 2005.

Thus, there is presently provided a method for delivering a neuroprotective agent to a neural cell, the method comprising contacting the neural cell with a compound of general formula I, a pharmaceutically acceptable salt thereof or an oligomer or polymer thereof. As used herein, "delivering" a neuroprotective agent to a neural cell refers to providing the agent in sufficiently close proximity to the cell such that the agent can exert its neuroprotective effects on the cell. *In vitro*, for example, the agent may be delivered to the neural cell by adding the agent to the cell culture media. *In vivo*, for example, the agent may be delivered by administering the agent to a subject as a pharmaceutical composition.

As used herein, "contacting" a cell refers to direct and indirect contact with the cell. Direct contact refers to a direct interaction between the agent and the cell. In contrast, indirect contact involves "contacting the cell" via interactions with other molecules or compounds. For example, the agent may interact with a molecule, effecting a change in that molecule that causes it to interact with the cell or interact with another molecule which will then interact with the cell to exert an effect. Indirect contact may involve a series of molecular interactions resulting in an effect on the cell.

A "neuroprotective agent" as used herein refers to a compound that alters, reduces, impairs or inhibits, partially or completely, molecules, mechanisms or effects associated with neurological disorders. A neuroprotective agent may, for example, reduce or inhibit damage to neural cells including for example, loss of neurons and gliosis. Without being limited to any particular theory, the neuroprotective agents described herein may exert their effects by reducing, impairing, inhibiting or altering, partially or completely, the harmful activity of neurotoxic molecules or mechanisms, by increasing the resistance of neural cells to neurotoxic molecules or mechanisms or by both reducing harmful activity and increasing neural cell resistance.

As used herein, "neural cell" refers to neurons, glial cells, oligodendricytes and microglia. Neural cells may be found in the central nervous system including regions of the brain and spinal cord, the peripheral nervous system, the retina and the optic nerve. A neural cell may include, for example, a retinal ganglion cell, a cell of the SNpc, a cell of the striatum, a dopaminergic neuron, a neuron of the nigrostriatal DA pathway, an astrocyte, a Muller cell, a Schwann cell or a motor neuron.

The neural cell to which the neuroprotective agent is to be provided may be any neural cell, including an *in vitro* neural cell, a neural cell in culture, or an *in vivo* neural cell within a subject. The term "cell" as used herein refers to and includes a single cell, a plurality of cells or a population of cells where context permits, unless otherwise specified. The cell may be an *in vitro* cell including a cell explanted from a subject or it may be an *in vivo* cell in a subject. Similarly, reference to "cells" also includes reference to a single cell where context permits, unless otherwise specified. In particular embodiments, the neural cell to which the neuroprotective agent is provided is, for example, a neuron or a glial cell.

The neural cell may be derived from any organism that has a neural cell, for example an insect or an animal including a mammal including a human.

The neural cell of the present method may be within a subject having a neurological disorder, a subject requiring treatment for a neurological disorder or a subject in which prevention of a neurological disorder is desired. In some embodiments, the subject is a human subject.

Thus, an effective amount of a neuroprotective agent may be delivered to a cell in a subject for the treatment of a neurological disorder.

"Neurological disorder" will be understood by those skilled in the art to refer to a disease or injury of the central nervous system characterized or caused by damaged, defective, malfunctioning or deficient neural cells. Neurological disorders may include for example, stroke, ischemia, epilepsy, Parkinson's disease, Huntington's disease, Alzheimer's disease, multiple sclerosis, amyotrophic lateral sclerosis, neurogenetic disorders, head and spinal cord trauma or injury, drug-induced neurotoxicity, toxin-induced neurotoxicity, chemical induced neurotoxicity, pesticide-induced neurotoxicity, eye injury and retinopathy.

The neurological disorder, in a particular embodiment is Parkinson's disease. In another embodiment, the neurological disorder is retinopathy.

The neuroprotective agent may be for example 1,3-bisbenzylimidazolium or a pharmaceutically acceptable salt thereof.

In another embodiment, the neuroprotective agent may be for example 1,3-diisopropylimidazolium or a pharmaceutically acceptable salt thereof.

The term "effective amount" as used herein means an amount effective, at concentrations, dosages and/or periods of time necessary to achieve the desired result, for example to provide a neuroprotective effect or to treat a neurological disorder. The total amount of IMS to be administered will vary, depending on several factors, including the severity and type of the disorder, the mode of administration, and the age and health of the subject. Methods for determining an effective amount of a particular IMS for treating a neurological disorder will be readily apparent to a person skilled in the art.

"Treating" neurological disorders refers to an approach for obtaining beneficial or desired results, including clinical results. Beneficial or desired clinical results can include, but are not limited to, alleviation or amelioration of one or more symptoms or conditions, diminishment of extent of disorder or disease, stabilization of the state of disease, prevention of development of disorder or disease, prevention of spread of disorder or disease, delay or slowing of disorder or disease progression, delay or slowing of disorder or disease onset, amelioration or palliation of the disorder or disease state, and remission, whether partial or total. "Treating" can also mean prolonging survival of a subject beyond that expected in the absence of treatment. "Treating" can also mean inhibiting the progression of disorder or disease, slowing the progression of disorder or disease temporarily, although in some instances, it involves halting the progression of the disorder or disease permanently.

To aid in administration to a subject, the IMS may be formulated as an ingredient in a pharmaceutical composition. The compositions may contain pharmaceutically acceptable concentrations of salt, buffering agents, preservatives and various compatible carriers or diluents.

The proportion and identity of the pharmaceutically acceptable carrier is dependant on a variety of factors including the chosen route of administration, compatibility with the IMS molecule and standard pharmaceutical practice. Generally, the pharmaceutical composition will be formulated with components that will not significantly impair the biological properties of the IMS.

Suitable vehicles and diluents are described, for example, in Remington's Pharmaceutical Sciences (Remington, The Science and Practice of Pharmacy, 21st edition, Lippincott Williams & Wilkins, Philadelphia, PA., 2006). It would be known to a person skilled in the art how to prepare a suitable pharmaceutical composition.

The pharmaceutical composition may be administered to a subject in a variety of forms depending on the selected route of administration, as will be understood by those skilled in the art. The composition of the invention may be administered for example, by oral administration, surgically or by injection to the desired site.

In different embodiments, the composition is administered by injection (subcutaneously, intravenously, intramuscularly, etc.) directly at a desired site, for example in the vicinity of the neurological disorder that is to be treated.

The dose of the pharmaceutical composition that is to be used depends on the particular neurological disorder being treated, the severity of the condition, individual patient parameters including age, physical condition, size and weight, the duration of the treatment, the nature of concurrent therapy (if any), the specific route of administration and other similar factors that are within the knowledge and expertise of the health practitioner. These factors are known to those of skill in the art and can be addressed with minimal routine experimentation.

It will be understood that pharmaceutical compositions may be provided in a variety of dosage forms and thus, in different embodiments, IMSs may be administered in different dosage forms including for example pills, tablets, capsules, solutions, suspensions, powder and injections. Conventional procedures and ingredients for preparing and administering the different dosage forms would be known to a skilled person and are described for example, in Remington's Pharmaceutical Sciences (Remington, The Science and Practice of Pharmacy, 21st edition, Lippincott Williams & Wilkins, Philadelphia, PA., 2006).

A skilled person will understand that a neuroprotective agent described herein may be administered as part of a combined therapy with another compound for the treatment neurological disorders. For example, the neuroprotective agent may be provided in combination with a known drug for the treatment of neurological disorders currently in clinical use. Combined therapy may involve simultaneous or sequential administration of the compounds included in the therapy.

Uses of the IMSs as described herein, including compounds having a structure of general formula I, a pharmaceutically acceptable salt thereof or an oligomer or polymer thereof for delivering a neuroprotective agent to a neural cell and in the manufacture of a medicament for delivering a neuroprotective agent to a neural cell are also contemplated. Also contemplated are uses of the IMSs as described herein for treatment of a neurological disorder in a subject, or in the manufacture of a medicament for treatment of a neurological disorder in a subject.

The present inventors have discovered that the IMSs described herein have a neuroprotective effect, which may include inhibiting gliosis in the retina and attenuating DA neuron loss in the SNpc. Without being limited to any particular theory, the IMSs may protect neural cells by efficiently passing through the blood-retinal barrier and the blood-brain-barrier and penetrating glial and neuronal cells where they exert anti-neurotoxic and neuroprotective properties. Alternatively, the IMSs may exert their neuroprotective effects on neural cells indirectly, by interacting with molecules, compounds or other cells to affect a change in those molecules, compounds or cells that in turn exert neuroprotective properties.

The IMSs used in the present method may exert their neuroprotective effect as a result of anti-oxidative properties, which may function via a multitude of molecular interactions. Anti-oxidants generally exert their effect mainly through three different pathways: (1) neutralization of cellular free radical ROS generated during metabolism and immune response, (2) induction of endogenous anti-oxidative enzymatic activity, and (3) chelation of iron or copper ions that catalyze the generation of hydroxyl radical. Without being limited to any particular theory, it appears that the anti-oxidative properties of the IMSs may result, at least in part, due to their activity as radical scavengers. It appears that the neutralization of free radicals by IMSs may occur after a series of chemical reactions including (1) the spontaneous conversion of IMSs to NHCs preferably under a basic condition, (2) the interaction of the NHCs at the carbon 2-position with free radicals such as ROS resulting in the formation of intermediate active radicals and the neutralization of the free radicals.

In addition to anti-oxidative mechanisms, there may be other mechanisms by which the IMSs presently provided may exert their neuroprotective effects. For example, the IMSs may exert neuroprotective effects through anti-inflammatory properties. Furthermore, the mechanisms by which the IMSs exert their neuroprotective effects may be multi-factorial. For example, IMSs may reduce, inhibit or modify the activity or expression of various molecules involved in inflammation, including for example the activator protein-1 (AP-1), nuclear factor kappa B (NF-κB) and TNF-α. TNF-α is produced primarily in macrophages and plays a major role in the pathogenesis of inflammation. It is known that the binding of TNF-α to its membrane receptor TNF-R1 activates multiple downstream signalling events, including activation of caspases and NF-κB-mediated transcription (Cleren et al., 2005)

The present methods and compounds are further exemplified by way of the following non-limited examples.

### EXAMPLES

### Materials and Methods

### Transgenic GFAP-GFP mice

The generation and genotyping of the transgenic GFAP (glial fibrillary acidic protein)-GFP (green fluorescent protein) mice were performed as previously described (Zhuo et al., 1997, See PCT Application No. PCT/SG2008/000159). Adult mice (8-10 weeks old) in the Friend virus B-Type (FVB/N) background were used in this study. Animal husbandry was provided by the Biological Resource Center (BRC) of Biopolis, Singapore. The experimental protocol for this study was approved by the Institutional Animal Care and Use Committee at BRC.

### DBZIM, neurotoxicant, and dosing regime

The synthesis of DBZIM has been described. (See PCT Application No. SG2009/000037, Zhang et al., 2007). The structure of DBZIM is illustrated in Fig 1. The neurotoxicant methyl-4(2'-methylphenyl)-1,2,3,6-tetrahydropyridine (2'-CH₃-MPTP), which is a more potent analog of MPTP for inducing Parkinson's disease in the adult brain (Abdel-Wahab, 2005), was purchased from Sigma-Afdrich (M103; St. Louis, MO, USA). The dosing regime is illustrated in Fig. 2. Mice in the DBZIM/neurotoxicant-treated group received an intraperitoneal (ip) injection of DBZIM (25 mg/kg in saline) 24 h before and after administering 4 ip injections of 2'-CH₃-MPTP (15 mg/kg in saline; once every 2 h). Mice in the neurotoxicant-treated group were injected with 2'-CH₃-MPTP only (4 × 15 mg/kg in saline; ip; once every 2 h). For each mouse in the DBZIM/neurotoxicant-treated group (*n* = 5) and neurotoxicant-treated group (*n* = 5), saline was used as a vehicle for the control group (*n* = 6). Retinal imaging was performed before and after treatment.

### Preparation of animals

Mice were anesthetized by ip injections with 0.15 ml/10 g body weight of Avertin (1.5% 2,2,2-tribromoethanol; T48402) purchased from Sigma-Aldrich (St. Louis, MO, USA). Their pupils were dilated with a drop of 0.5% Cyclogyl^{®} sterile ophthalmic solution (cyclopentolate hydrochloride, Alcon^{®}, Puurs, Belgium). Custom-made polymethylmethacrylate (PMMA) hard contact lenses (Cantor & Nissel, Northamptonshire, UK) were used to avoid dehydration of the cornea. Careful examination by an eye specialist before scanning laser ophthalmoscope (SLO) imaging ruled out the presence of any corneal or lens opacity.

### Scanning Laser Ophthalmoscope (SLO imaging)

The second version of Heidelberg Retina Angiograph (HRA II) SLO (Heidelberg Engineering, Dossenheim, Germany) was modified for use on mice. SLO is an imaging technique based on the scanning of the fundus with a laser beam from a point source; the reflected light is detected by a photomultiplier. The incident and reflected light beams follow a co-axial path. Therefore, more light can pass through small eyes with SLO than with conventional fundus cameras. Several reports of SLO imaging of rats (Hossain et al., 1998; Khoobehi and Peyman, 1999; Cordeiro et al., 2004; Genevois et al., 2004) and mice (Jaissle et al., 2001; Xu et al., 2003) have been published. The latest studies (Seeliger et al., 2005; Paques et al., 2006) described evaluating mouse fundus with the first version of the HRA (HRA I). The HRA II features two argon lasers in the short wavelength range (488 nm and 514 nm), and two infrared diode lasers in the long wavelength range (795 nm and 830 nm). The 488-nm and 795-nm lasers were used for fluorescein and indocyanine green angiography, respectively. Appropriate barrier filters of 500 nm and 800 nm, respectively, were employed to remove the reflected light with unchanged wavelength, and allowed only the light emitted by the dye upon stimulation to pass through. The finest definition was 768 × 768 pixels at an optical resolution of 10 µm/pixel, coupled with three fields of view (with nominal values of 15°, 20° and 30°). The focus was adjustable over a +12/-12 diopters range using step increments of 0.25 diopters. A video acquisition mode (48-96 ms/image) was available. Within any area of interest, a stack of tomographic images (z-scans) up to a maximum depth of 8 mm could be automatically acquired.

### Image processing and quantification

Unlike magnetic resonance images (MRI) and *in vitro* fluorescentlabeled cell images, the *in vivo* fluorescence images of transgenic GFAP-GFP expression in the mouse retina have very low signal-to-noise ratio (SNR), which makes the quantitative analysis of the transgene expression difficult. To overcome this challenge, an algorithm was developed to automatically align multiple images collected from different XYZ positions to achieve higher SNR and resolution for the final composite image. The details of the algorithm development are described separately (Kumar et al., 2007). All the image processing and signal quantification were performed using the algorithm developed. The quantified values were expressed as mean ± standard error of mean (SEM), and statistically tested using Student's two-tailed t-test.

### Retinal whole-mount and immunohistochemistry (IHC)

After perfusion with 1× PBS, followed by 4% fresh paraformaldehyde (in 1 × PBS, pH 7.4), the mouse brains were harvested and the eyes enucleated. The eyes were immediately fixed in 4% paraformaldehyde overnight at 4°C, after which they were dissected at the equator with the lens and vitreous removed. The retinas, free of the choroid and sclera tissues, were whole-mounted on a glass slide with Vectorshield mounting medium for fluorescence (Vector Laboratories, Inc., Burlingame, CA, USA; H1000). The brains were first fixed in 4% paraformaldehyde at 4°C for 4 h, and soaked in 30% sucrose at 4°C overnight. The processed brain tissues were embedded in OTC freezing medium (Fisher Scientific, Pittsburgh, PA, USA) for sectioning on a cryostat (Leica Microsystems, Nussloch GmbH; CM-3050S). The retina whole-mounts were used for IHC using a rabbit polyclonal antibody against GFAP (Z0334; Dako, Carpinteria, CA, USA) in 1:200 dilution. Coronal cryosections (5 µm) of the SNpc (bregma -3.16 mm, interaural 0.64 mm) and striatum (bregma 0.62 mm, interaural 4.42 mm) according to the Atlas of Mouse Brain (Franklin and Paxinos, 2001) were used for IHC using a rabbit polyclonal antibody against tyrosine hydroxylase (TH) (Chemicon, CA, USA; Ab-152) in 1:200 dilution. The bound primary antibodies on the tissue sections were stained with a goat IgG conjugated to Texas-red (Abcam, Ab7088) at 1:100 dilution at room temperature for 2 h, and visualized using confocal microscopy (LSM 510 META, Carl Zeiss Microimaging GmbH, Jena, Germany).

### Results

### DBZIM reduced 2'-CH₃-MPTP-induced retinal neurotoxicity as revealed by quantitative retinal imaging

Representative composite retinal images for mice from different groups (saline control, 2'-CH₃-MPTP-treated and DBZIM/2'-CH₃-MPTP-treated) are shown in Fig. 3. It is clear that the neurotoxicant 2'-CH₃-MPTP causes a drastic increase in GFP fluorescence on the mouse optic disc two days post-dosing (Fig. 3, middle column). Regions with the highest GFP fluorescence were mainly located in the fringe of the optic disc where axon fibers and retinal vasculature were congregated with retinal glia. In contrast, mice injected with DBZIM and 2'-CH₃-MPTP separately displayed no apparent neurotoxicity (Fig. 3, right column). Images acquired directly from a particular retina of a living mouse have their distinct X-Y-Z positions, primarily due to the continuous motion of breathing and heartbeat of the anesthetized mouse. Therefore, the stack of images could not be simply overlaid and averaged to obtain a final composite image with sufficient resolution for analysis/quantification using the built-in program in the HRA II SLO. To accurately quantify the GFP fluorescence intensity from stacks of raw retinal images, a proprietary algorithm was developed. The detailed development and additional applications of this algorithm are described elsewhere (Saravana et al., 2007). Using this algorithm for quantification, a 36% increase in the GFP fluorescence was obtained for the optic disc in mice administered with the neurotoxicant alone (Fig. 4). In contrast, mice injected with DBZIM and the neurotoxicant showed no significant change in the GFP fluorescence at a basal level comparable to that of the saline-treated control mice (Fig. 4), suggesting a significant attenuation in retinal neurotoxicity.

### Retinal IHC data confirmed in vivo imaging observations

To prove that the green fluorescence signal detected from the retina is indeed from the retinal glia, and not from other retinal cell types or the plasma within the retinal blood vessels, IHC was performed on the fixed retinas of the mice subjected to the three treatments. Tiled confocal images of GFAP immunostained (red) retinal whole-mounts from the saline-treated, neurotoxicant-treated and DBZIM/neurotoxicant-treated adult mice were examined. Following the administration of the neurotoxicant, severe gliosis evidenced by an elevated fluorescence for the transgenic GFP (green) and for the endogenous GFAP (red) in the nerve fiber layer (NFL) could be seen in the optic disc and peripheral retina, when compared to the saline control (Fig. 5). As expected, DBZIM attenuated both green and red fluorescence to the basal levels similar to the saline control group (Fig. 5). In the cross-sections of the saline-treated retina, the Müller cells with their cell bodies located in the inner nuclear layer (INL) and the cellular processes extending radially throughout almost the entire retina were prominently labeled with GFP, but with very little or no GFAP staining under our image acquisition settings (Fig. 6). In contrast, the neurotoxicant induced severe gliosis in both the Müller cells and astrocytes at the GCL (Ganglion Cell Layer), whereas the DBZIM reduced the neurotoxicant-induced gliosis to close to the basal level seen in the saline control (Fig. 6).

### DBZIM reduced 2'-CH₃-MPTP-induced striatal gliosis and DA neuronal depletion in the SNpc,

It was well established that MPTP and its analog trigger striatal gliosis as measured by using GFAP antibody. Herein, it was shown that the GFAP-GFP transgene (as a GFAP surrogate) responded similarly to 2'-CH₃-MPTP by up-regulating its expression. Interestingly, treatment with DBZIM again cut down the gliosis in the striatum to a basal level as in the saline control (Fig. 7). In the SNpc, DBZIM not only attenuated gliosis, but also protected a significant portion of the DA neurons (as marked by the TH (tyrosine hydroxylase)-immunoreactivity in red) from depletion by 2'-CH₃-MPTP (Fig. 8).

. All publications and patent applications cited in this specification are herein incorporated by reference as if each individual publication or patent application were specifically and individually indicated to be incorporated by reference. The citation of any publication is for its disclosure prior to the filing date and should not be construed as an admission that the present invention is not entitled to antedate such publication by virtue of prior invention.

Concentrations given in this specification, when given in terms of percentages, include weight/weight (w/w), weight/volume (w/v) and volume/volume (v/v) percentages.

As used in this specification and the appended claims, the singular forms "a", "an" and "the" include plural reference unless the context clearly dictates otherwise. As used in this specification and the appended claims, the terms "comprise", "comprising", "comprises" and other forms of these terms are intended in the non-limiting inclusive sense, that is, to include particular recited elements or components without excluding any other element or component. Unless defined otherwise all technical and scientific terms used herein have the same meaning as commonly understood to one of ordinary skill in the art to which this invention belongs.

### References

Abdel-Wahab, M H, 2005. Potential neuroprotective effect of t-butylhydraquinone against neurotoxicity induced by 1-methyl-4-(2'-methylphenyl)-1,2,3,6-tetrahydropyridine (2'-methyl-MPTP) in mice. J. Biochem. Mol. Toxicol. 19, 32-41.
Bezard, E, Gerlach, I, Moratalla, R, Gross, C E, Jork, R, 2006. 5-HT1A receptor agonist-mediated protection from MPTP toxicity in mouse and macaque models of Parkinson's disease. Neurobiol. Dis. 23, 77-86.
Blanks, J C, Schmidt, S Y, Torigoe, Y, Porrello, K V, Hinton, D R, Blanks, R H., 1996. Retinal pathology in Alzheimer's disease. II. Regional neuron loss and glial changes in GCL. Neurobiol. Aging. 17, 385-395.
Bodis-Wollner, I, 1990. Visual deficits related to dopamine deficiency in experimental animals and Parkinson's disease patients. Trends Neurosci. 13, 296-302.
Bove, J, Prou, D, Perier, C, Przedborski, S, 2005. Toxin-induced models of Parkinson's disease. NeuroRX 2, 484-494.
Boydston A. J. , Williams K. A., Bielawski C. W., J. Am. Chem. Soc., 2005, 127, 12496.
Brenner, M, Kisseberth, W C, Su, Y, Besnard, F, Messing, A, 1994. GFAP promoter directs astrocyte-specific expression in transgenic mice. J. Neurosci. 14, 1030-1037.
Casanovas A, Olmos G, Ribera J, Boronat M A, Esquerda J E, Garcia-Sevilla J A. Induction of reactive astrocytosis and prevention of motoneuron cell death by the I2-imidazoline receptor ligand LSL 60101. Br J Pharmacol 2000;130:1767-1776.
Chianese R. A. and Cratree R. H. Organometallics, 2005, 24, 4432.
Chen, S T, Hsu, J R, Hsu, P C, Chuang, J I, 2003. The retina as a novel in vivo model for studying the role of molecules of the Bcl-2 family in relation to MPTP neurotoxicity. Neurochem. Res. 28, 805-814.
Choi, J Y, Park, C S, Kim, D J, Cho, M H, Jin, B K, Pie, J E, Chung, W G, 2002. Prevention of nitric oxide-mediated 1-methyl-4-phenyl-1,2,3,6-tetrahydropyridine-induced Parkinson's disease in mice by tea phenolic epigallocatechin 3-gallate. Neurotoxicology 23, 367-374.
Cleren, C, Calingasan, N Y, Chen, J, Beal M F, 2005. Celastrol protects against MPTP- and 3-nitropropionic acid-induced neurotoxicity. J. Neurochem. 94, 995-1004.
Cordeiro, M F, Guo, L, Luong, V, Harding, G, Wang, W, Jones, H E, Moss, S E, Sillito, A M, Fitzke, F W, 2004. Real-time imaging of single nerve cell apoptosis in retinal neurodegeneration. Proc. Natl. Acad. Sci. U.S.A. 101, 13352-13356.
Dauer, W, Przedborski, S, 2003. Parkinson's disease: Mechanisms and models. Neuron. 39, 889-909.
Dyer, M A, Cepko, C L, 2000. Control of Müller glial cell proliferation and activation following retinal injury. Nat. Neurosci. 3, 873-880.
Franklin, K B J, Paxinos, G, 2001. The mouse brain in stereotaxic coordinates. Academic Press, London.
Genevois, O, Paques, M, Simonutti, M, Sercombe, R, Seylaz, J, Gaudric, A, Brouland, J P, Sahel, J, Vicaut, E, 2004. Microvascular remodeling after occlusion-recanalization of a branch retinal vein in rats. Invest. Ophthalmol. Vis. Sci. 45, 594-600.
Grunblatt, E, Mandel, S, Youdim, M B, 2000. MPTP and 6-OHDA-induced neurodegeneration as models for Parkinson's disease. J. Neurol. 247 (Suppl II), 95-102.
Harlow K J, Hill A F, Welton T, Convenient and general synthesis of symmetric N,N'-disubstituted imidazolium halides, Synthesis, 1996;6:697-698.
Helmlinger, D, Yvert, G, Picaud, S, Merienne, K, Sahel, J, Mandel, J L, Devys, D, 2002. Progressive retinal degeneration and dysfunction in R6 Huntington's disease mice. Hum. Mol. Genet. 15, 3351-3359.
Hirsch, E C, Hunot, S, Hartmann, A, 2005. Neuroinflammatory processes in Parkinson's disease. Parkinsonism Relat. Disord. 11, 3-15.
Ho, G, Zhang, C Y, Zhuo, L, 2007. Non-invasive fluorescent imaging of gliosis in transgenic mice for profiling developmental neurotoxicity. Toxicol. Appl. Pharmacol. doi:10.1016/j.taap.2007.01.023 (in press).
Hossain, P, Liversidge, J, Cree, M J, Manivannan, A, Vieira, P, Sharp, P F, Brown, G C, Forrester, J V, 1998. In vivo cell tracking by scanning laser ophthalmoscopy: Quantification of leukocyte kinetics. Invest. Ophthalmol. Vis. Sci. 39, 1879-1887.
Jaissle, G B, May, C A, Reinhard, J, Kohler, K, Fauser, S, Lütjen-Drecoll, E, Zrenner, E, Seeliger, M W, 2001. Evaluation of the rhodopsin knockout mouse as a model of pure cone function. Invest. Ophthalmol. Vis. Sci. 42, 506-513.
Jellinger, K A, 1999. The role of iron in neurodegeneration: Prospects for pharmacotherapy of Parkinson's disease. Drugs Aging 14, 115-140.
Khoobehi, B, Peyman, G A, 1999. Fluorescent labeling of blood cells for evaluation of retinal and choroidal circulation. Ophthalmic Surg. Lasers 30, 140-145.
Kumar, S, Ho, G, Zhuo, L, 2007. Non-linear motion compensation for automated frame averaging of noisy in vivo images acquired from the retinas of transgenic GFP mice (in preparation).
Kuzmanovic, M, Dudley, V J, Sarthy, V P, 2003. GFAP promoter drives Müller cell-specific expression in transgenic mice. Invest. Ophthalmol. Vis. Sci. 44, 3646-3613.
Levites, Y, Weinreb, O, Maor, G, Youdim, M B H, Mandel, S, 2001. Green tea polyphenol (-)-epigallocatechin-3-gallate prevents 1-methyl-4-phenyl-1,2,3,6-tetrahydropyridine-induced dopaminergic neurodegeneration. J. Neurochem. 78, 1073-1082.
Marin, C, Bove, J, Serrats, J, Cortes, R, Mengod, G, Tolosa, E, 2005. The kappa opioid agonist U50,488 potentiates 6-hydroxydopamine-induced neurotoxicity on dopaminergic neurons. Exp. Neurol. 191, 41-52.
Mogi, M, Harada, M, Kondo, T, Riederer, P, Inagaki, H, Minami, M, Nagatsu, T, 1994a. Interleukin-1β, interleukin-6, epidermal growth factor and transforming growth factor-a are elevated in the brain from Parkinsonian patients. Neurosci. Lett. 180, 147-150.
Mogi, M, Harada, M, Riederer, P, Narabayashi, H, Fujita, K, Nagatsu, T, 1994b. Tumor necrosis factor-α (TNF-α) increases both in the brain and in the cerebrospinal fluid from Parkinsonian patients. Neurosci. Lett. 165, 208-210.
Morgan, J, Huckfeldt, R, Wong, R O, 2005. Imaging techniques in retinal research. Exp. Eye Res. 80, 297-306.
Olanow, W, Tatton, W G, 1999. Etiology and pathogenesis of Parkinson's disease. Annu. Rev. Neurosci. 22, 123-144.
Olmos G, DeGregorio-Rocasolano N, Regalado M P, Gasull T, Boronat M A, Trullas R, Villarroel A, Lerma J, Garcia-Sevilla J A. Protection by imidazol(ine) drugs and agmatine of glutamate-induced neurotoxicity in cultured cerebellar granule cells through blockade of NMDA receptor. Br J Pharmacol 1999;127:1317-1326.
Paques, M, Simonutti, M, Roux, M J, Picaud, S, Levavasseur, E, Bellman, C, Sahel, J A, 2006. High resolution fundus imaging by confocal scanning laser ophthalmoscopy in the mouse. Vision Res. 46, 336-1345.
Peng, J, Stevenson, F F, Doctrow, S R, Andersen, J K, 2005. Superoxide dismutase/catalase minetics are neuroprotective against selective paraquat-mediated dopaminergic neuron death in the substantia nigra: Implications for Parkinson disease. J. Biol. Chem. 280, 29194-29198.
Peters, S, Schweibold, G, Przuntek, H, Muller, T, 2000. Loss of visual acuity under dopamine substitution therapy. Neuro-ophthalmology. 24, 273-277.
Przedborski, S, Jackson-Lewis, V, Naini, A, Jakowec, M, Petzinger, G, Miller, R, 2001. The Parkinsonian toxin 1-methyl-4-phenyl-1,2,3,6-tetrahydropyridine (MPTP): A technical review of its utility and safety. J. Neurochem. 76, 1265-1274.
Przedborski, S, Vila, M, 2001. MPTP: A review of its mechanisms of neurotoxicity. Clin. Neurosci. Res. 1, 407-418.
Seeliger, M W, Beck, S C, Pereyra-Munoz, N, Dangel, S, Tsai, J Y, Luhmann, F O, 2005. In vivo confocal imaging of the retina in animal models using scanning laser opthalmoscopy. Vision Res. 45, 3512-3519.
Smeyne, R J, Jackson-Lewis, V, 2005. The MPTP model of Parkinson's disease. Mol. Brain Res. 134, 57-66.
Wu, D C, Jackson-Lewis, V, Vila, M, Tieu, K, Teismann, P, Vadseth, C, Choi, D K, Ischiropoulos, H, Przedborski, S, 2002. Blockade of microglial activation is neuroprotective in the 1-methyl-4-phenyl-1,2,3,6-tetrahydropyridine mouse model of Parkinson disease. J. Neurosci. 22, 1763-1771.
Xu, H, Manivannan, A, Liversidge, J, Sharp, P F, Forrester, J V, Crane, I J, 2003. Requirements for passage of T lymphocytes across non-inflamed retinal microvessels. J. Neuroimmunol. 142, 47-57.
Zhang Y, Ngeow K. C. and Ying J. Y. Organic Letters, 9 [18] (2007) 3495-3498.
Zhang, C Y, Zhang, Y G, Ying, J Y, Zhuo, L, 2007. A novel class of synthetic imidazolium salts displaying anti-oxidative, anti-inflammatory and anti-fibrotic effects in hepatic stellate cells. To be submitted.
Zhuo, L, Sun, B, Zhang, C L, Fine, A, Chiu, S Y, Messing, A, 1997. Live astrocytes visualized by green fluorescent protein in transgenic mice. Dev. Biol. 187, 36-42.

The following numbered paragraphs (paras.) contain statements of broad combinations of the inventive technical features herein disclosed:-
1. A method for delivering a neuroprotective agent to a neural cell, the method comprising contacting the neural cell with a compound of general formula I or an oligomer or polymer thereof: 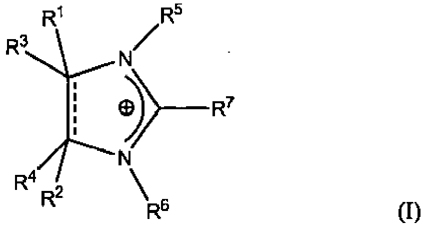 wherein:
   the dashed line is absent or is present as a bond to form a second bond between the carbon to which R¹ and R³ are attached and the carbon to which R² and R⁴ are attached;
   R¹ and R²:
   (i) are each independently H, straight or branched C₁-C₆ alkyl, straight or branched C₂-C₆ alkenyl, straight or branched C₂-C₆ alkynyl, C₆-C₁₀ acryl;
   (ii) together with their central ring atoms form a 6- to 10-membered fused saturated, unsaturated or aromatic ring system;
   (iii) R¹ and R⁵ together with their central ring atoms, or R² and R⁶ together with their central ring atoms, form a 5- to 10-membered fused saturated, unsaturated or aromatic ring system and the other of R¹ and R² is as defined above in (i); or
   (iv) R¹ and R⁵ together with their central ring atoms and R² and R⁶ together with their central ring atoms each independently form a 5- to 10-membered fused saturated, unsaturated or aromatic ring system;
   R³ and R⁴ are both H, or, when R¹ and R² together with their central ring atoms form a 6- to 10-membered fused aromatic ring system or when the dashed line is present as a bond, R³ and R⁴ are absent;
   R⁵ or R⁶:
   (i) are as defined above for R¹ and R²; or
   (ii) are each independently straight or branched C₁-C₆ alkyl, straight or branched C₂-C₆ alkenyl, straight or branched C₂-C₆ alkynyl, C₃-C₁₈ cycloalkyl including fused cycloalkyl ring systems, C₆-C₁₀ aryl, C₆-C₁₀ aryl-C₁-C₆ alkyl, C₆-C₁₀ aryl-C₂-C₆ alkenyl, or C₆-C₁₀ aryl-C₂-C₆ alkynyl, C₁-C₆ alkyl-C₆-C₁₀ aryl, C₂-C₆ alkenyl-C₆-C₁₀ aryl, or C₂-C₆ alkynyl-C₆-C₁₀ aryl;
   R⁷ is H, C₁-C₆ alkyl or C₆-C₁₀ aryl;
   in which any of R¹ to R⁶, where applicable, optionally has one or more carbon atoms replaced with a heteroatom selected from N, O, S and P and is optionally substituted with one or more of straight or branched C₁-C₆ alkyl, straight or branched C₂-C₆ alkenyl, straight or branched C₂-C₆ alkynyl, C₃-C₁₈ cycloalkyl including fused cycloalkyl ring systems, C₆-C₁₀ aryl, fluoro, tri-fluoro-methyl, cyanato, isocyanato, carboxyl, C₁-C₆ acyloxy, C₁-C₆ acyl, carbonyl, amino, acetyl, acetoxy, oxo, nitro, hydroxyl, C₁-C₆ alkylcarboxy, C₁-C₆ alkoxy, C₂-C₆ alkenoxy, C₂-C₆ alkynoxy; and
   in which one of the central ring carbon atom to which R¹ and R³ are attached and the central ring carbon to R² and R⁴ are attached is optionally replaced with a nitrogen atom;
   or a pharmaceutically acceptable salt of the compound or of the oligomer or polymer of the compound.
2. The method of para. 1 wherein the compound is an imidazolium or an imidazolium salt.
3. The method of para. 1 wherein the compound is an imidazolinium or an imidazolinium salt.
4. The method of para. 1 wherein R⁵ is the same as R⁶.
5. The method of para. 1 wherein R⁵ and R⁶ are hydrocarbons.
6. The method of para. 1 wherein the pharmaceutically acceptable salt is a chloride, bromide, tetrafluoroborate or hexafluorophosphate salt.
7. The method of para. 1 wherein R⁷ is H.
8. The method of para. 1 wherein the compound is 1-ethyl-3-methylimidazolium, 1,3-bisbenzylimidazolium, 1,3-diisopropylimidazolium, 1,3-di-tert-butylimidazolinium, 1,3-bis(1-adamantyl)imidazolium, 1,3-bis(2,4,6-trimethylphenyl)-imidazolinium, 1,3-bis(2,6-diisopropyl-phenyl)-imidazolinium, 1,3,-bisbenzylbenzimidazolium, 1,3-dibenzyl-2-methylimidazolium, 1,3-diallylimidazolium, 1-benzyl-3-methylimidazolium, 1-butyl-3-methylimidazolium, 1-(1-adamantyl)-3-(2,4,6-trimethylphenyl)-4,5-dihydroimidazolium, 2-benzylimidazo[1,5-α]quinolinium, 1,3-bis(1-adamantyl)-benzimidazolium, 1,3-dicyclohexylbenzimidazolium, 1,3-diisopropylimidazolinium tetrafluoroborate, 1,3-diisopropylimidazolium, 2-(2,6-diisopropylphenyl)-5-methylimidazo[1,5-α]pyridinium, 1-(2,6-diisopropylphenyl)-3-(2,4,6-trimethylphenyl)-imidazolinium, 2-mesityl-5-methylimidazo[1,5-α]pyridinium, 2-mesityl-2,5,6,7-tetrahydropyrrolo[2,1-*c*][1,2,4]triazol-4-ium, 1,3-bis(1-adamantyl)imidazolinium, 1-butyl-3-(2-pyridinylmethyl)-1*H*-imidazolium, 6,7-dihydro-2-pentafluorophneyl-5H-pyrrolo[2,1-c]-1,2,4-trizolium, 1-methyl-3-(2-hydroxylethyl)-imidazolium, 1-methyl-3-(4-isocynatobenzyl)-imidazolium, 1-methyl-3-(4-carboxylbenzyl)-imidazolium, 1-methyl-3-(4-acetate-benzyl)-imidazolium, 1-methyl-3-(2,2-dimethoxylethyl)-imidazolium, 1-(2,4,6-trimethylphenyl)-3-(4-acetate-benzyl)-imidazolium, 1,3-Dibenzyl-5-phenylimidazolium, 1-benzyl-3-(4-carboxylbenzyl)-2-methylimidazolium, 1-benzyl-3-(3,4,5-trimethoxylbenzyl)-2-methylimidazolium, 1-benzyl-3-(4-acetate-benzyl)-2-methyl-imidazolium, 1-benzyl-3-(4-methylcarboxylatebenzyl)-2-methyl-imidazolium, 1-benzyl-3-(2,2-, dimethoxylethyl)-2-methyl-imidazolium, 2,6-di-(3-benzyl-imidazolium)-pyridine, 2,2'-di-(3-benzyl-imidazolium)-1,1'-binaphthalene,1-benzyl-3-(4-methylbenzyl)-2-methylimidazolium, 1-benzyl-3-(2-trifluoromethylbenzyl)-2-methylimidazolium, 1-benzyl-3-(4-methylcarboxylatebenzyl)-5-phenylimidazolium, 1-benzyl-3-(4-acetatebenzyl)-5-phenyl-imidazolium, 1-benzyl-3-(4-methylbenzyl)-5-phenylimidazolium, (1,24,5-diimidazolium)-N,N',N",N'''-tetrabenzyl-benzene, 1-benzyl-3-(2-propyn-1-yl)-imidazolium, 1-benzyl-3-(3-hydroxyl-propyl)-imidazolium, 1,3-di(2-phenylethyl)-imidazolium, 1-benzyl-3-(4-acetatebenzyl)-imidazolium, 1-benzyl-3-(pyridin-2-yl)-imidazolium, 1,3,5-tris-(4-methyl-imidazolium)-linked cyclophane, 1,3-dibenzyl-2-(1,3-dibenzyl-1H-imidazol-2(3H)-ylidene)-2,3-dihydro-1H-imidazole, 1-benzyl-3-methyl-imidazolium, 1-(4-cyanatobenzyl)-3-methyl-imidazolium, 1-(4-carboxybenzyl)-3-methyl-imidazolium, 1-methyl-3-(4-acetate-benzyl)-imidazolium, 1-methyl-3-(2,2-dimethoxyethyl)-imidazolium, or 1-(2,4,6-trimethylphenyl)-3-(4-acetatebenzyl)-imidazolium, or a pharmaceutically acceptable salt thereof.
9. The method of para. 1 wherein the compound is 1,3-bisbenzylimidazolium, 1,3-diisopropylimidazolium, 1,3-di-tert-butylimidazolinium, 1,3-bis(1-adamantyl)imidazolium, 1,3-bis(2,4,6-trimethylphenyl)-imidazolinium, 1,3-bis(2,6-diisopropyl-phenyl)-imidazolinium, 1,3,-bisbenzylbenzimidazolium, or 1,3,5-tris-(4-methyl-imidazolium)-linked cyclophane, or a pharmaceutically acceptable salt thereof.
10. The method of para. 1 wherein the compound is 1,3-bisbenzylimidazolium or a pharmaceutically acceptable salt thereof.
11. The method of para. 1 wherein the compound is 1,3-diisopropylimidazolium or a pharmaceutically acceptable salt thereof.
12. The method of para. 1 wherein the compound is a dimer of a compound having a structure of general formula I or a pharmaceutically acceptable salt thereof.
13. The method of para. 12 wherein the compound is 1,3-dibenzyl-2-(1,3-dibenzyl-1H-imidazol-2(3H)-ylidene)-2,3-dihydro-1H-imidazole, (1,2-4,5-diimidazolium)-N,N',N", N"'-tetrabenzyl-benzene, 2,6-di-(3-benzyl-imidazolium)-pyridine or 2,2'-di-(3-benzyl-imidazolium)-1,1'-binaphthalene, or a pharmaceutically acceptable salt thereof.
14. The method of para. 1 in which the compound is a trimer of a compound having a structure of general formula I or a pharmaceutically acceptable salt thereof.
15. The method of para.14 wherein the compound is 1,3,5-tris(4-methylimidazolium)-linked cyclophane or a pharmaceutically acceptable salt thereof.
16. The method of para. 1 wherein the cell is *in vitro*.
17. The method of para. 1 wherein the cell is *in vivo*.
18. The method of para. 17 wherein the method comprises administering an effective amount of the neuroprotective agent to a subject for the treatment of a neurological disorder.
19. The method of para. 18 wherein the neurological disorder is Parkinson's disease.
20. The method of para.18 wherein the neurological disorder is retinopathy.
21. Use of a compound for delivering a neuroprotective agent to a neural cell, the compound having a structure of general formula I or an oligomer or polymer thereof: 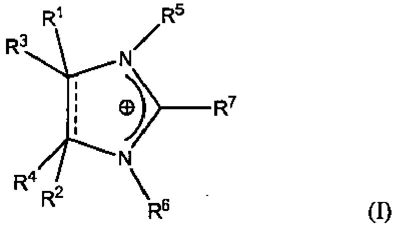 wherein:
   the dashed line is absent or is present as a bond to form a second bond between the carbon to which R¹ and R³ are attached and the carbon to which R² and R⁴ are attached;
   R¹ and R²:
   (i) are each independently H, straight or branched C₁-C₆ alkyl, straight or branched C₂-C₆ alkenyl, straight or branched C₂-C₆ alkynyl, C₆-C₁₀ aryl;
   (ii) together with their central ring atoms form a 6- to 10-membered fused saturated, unsaturated or aromatic ring system;
   (iii) R¹ and R⁵ together with their central ring atoms, or R² and R⁶ together with their central ring atoms, form a 5- to 10-membered fused saturated, unsaturated or aromatic ring system and the other of R¹ and R² is as defined above in (i); or
   (iv) R¹ and R⁵ together with their central ring atoms and R² and R⁶ together with their central ring atoms each independently form a 5- to 10-membered fused saturated, unsaturated or aromatic ring system;
   R³ and R⁴ are both H, or, when R¹ and R² together with their central ring atoms form a 6- to 10-membered fused aromatic ring system or when the dashed line is present as a bond, R³ and R⁴ are absent;
   R⁵ or R⁶:
   (i) are as defined above for R¹ and R²; or
   (ii) are each independently straight or branched C₁-C₆ alkyl, straight or branched C₂-C₆ alkenyl, straight or branched C₂-C₆ alkynyl, C₃-C₁₈ cycloalkyl including fused cycloalkyl ring systems, C₆-C₁₀ aryl, C₆-C₁₀ aryl-C₁-C₆ alkyl, C₆-C₁₀ aryl-C₂-C₆ alkenyl, or C₆-C₁₀ aryl-C₂-C₆ alkynyl, C₁-C₆ alkyl-C₆-C₁₀ aryl, C₂-C₆ alkenyl-C₆-C₁₀ aryl, or C₂-C₆ alkynyl-C₆-C₁₀ aryl;
   R⁷ is H, C₁-C₆ alkyl or C₆-C₁₀ aryl;
   in which any of R¹ to R⁶, where applicable, optionally has one or more carbon atoms replaced with a heteroatom selected from N, O, S and P and is optionally substituted with one or more of straight or branched C₁-C₆ alkyl, straight or branched C₂-C₆ alkenyl, straight or branched C₂-C₆ alkynyl, C₃-C₁₈ cycloalkyl including fused cycloalkyl ring systems, C₆-C₁₀ aryl, fluoro, tri-fluoro-methyl, cyanato, isocyanato, carboxyl, C₁-C₆ acyloxy, C₁-C₆ acyl, carbonyl, amino, acetyl, acetoxy, oxo, nitro, hydroxyl, C₁-C₆ alkylcarboxy, C₁-C₆ alkoxy, C₂-C₆ alkenoxy, C₂-C₆ alkynoxy; and
   in which one of the central ring carbon atom to which R¹ and R³ are attached and the central ring carbon to R² and R⁴ are attached is optionally replaced with a nitrogen atom;
   or a pharmaceutically acceptable salt of the compound or of the oligomer or polymer of the compound.
22. Use of a compound in the manufacture of a medicament for delivering a neuroprotective agent to a neural cell, the compound having a structure of general formula 1 or an oligomer or polymer thereof: 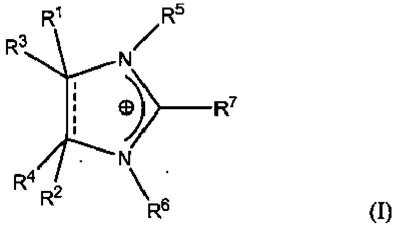 wherein:
   the dashed line is absent or is present as a bond to form a second bond between the carbon to which R¹ and R³ are attached and the carbon to which R² and R⁴ are attached;
   R¹ and R²:
   (i) are each independently H, straight or branched C₁-C₆ alkyl, straight or branched C₂-C₆ alkenyl, straight or branched C₂-C₆ alkynyl, C₆-C₁₀ aryl;
   (ii) together with their central ring atoms form a 6- to 10-membered fused saturated, unsaturated or aromatic ring system;
   (iii) R¹ and R⁵ together with their central ring atoms, or R² and R⁶ together with their central ring atoms, form a 5- to 10-membered fused saturated, unsaturated or aromatic ring system and the other of R¹ and R² is as defined above in (i); or
   (iv) R¹ and R⁵ together with their central ring atoms and R² and R⁶ together with their central ring atoms each independently form a 5- to 10-membered fused saturated, unsaturated or aromatic ring system;
   R³ and R⁴ are both H, or, when R¹ and R² together with their central ring atoms form a 6- to 10-membered fused aromatic ring system or when the dashed line is present as a bond, R³ and R⁴ are absent;
   R⁵ or R⁶:
   (i) are as defined above for R¹ and R²; or
   (ii) are each independently straight or branched C₁-C₆ alkyl, straight or branched C₂-C₆ alkenyl, straight or branched C₂-C₆ alkynyl, C₃-C₁₈ cycloalkyl including fused cycloalkyl ring systems, C₆-C₁₀ aryl, C₆-C₁₀ aryl-C₁-C₆ alkyl, C₆-C₁₀ aryl-C₂-C₆ alkenyl, or C₆-C₁₀ aryl-C₂-C₆ alkynyl, C₁-C₆ alkyl-C₆-C₁₀ aryl, C₂-C₆ alkenyl-C₆-C₁₀ aryl, or C₂-C₆ alkynyl-C₆-C₁₀ aryl;
   R⁷ is H, C₁-C₆ alkyl or C₆-C₁₀ aryl;
   in which any of R¹ to R⁶, where applicable, optionally has one or more carbon atoms replaced with a heteroatom selected from N, O, S and P and is optionally substituted with one or more of straight or branched C₁-C₆ alkyl, straight or branched C₂-C₆ alkenyl, straight or branched C₂-C₆ alkynyl, C₃-C₁₈ cycloalkyl including fused cycloalkyl ring systems, C₆-C₁₀ aryl, fluoro, tri-fluoro-methyl, cyanato, isocyanato, carboxyl, C₁-C₆ acyloxy, C₁-C₆ acyl, carbonyl, amino, acetyl, acetoxy, oxo, nitro, hydroxyl, C₁-C₆ alkylcarboxy, C₁-C₆ alkoxy, C₂-C₆ alkenoxy, C₂-C₆ alkynoxy; and
   in which one of the central ring carbon atom to which R¹ and R³ are attached and the central ring carbon to R² and R⁴ are attached is optionally replaced with a nitrogen atom;
   or a pharmaceutically acceptable salt of the compound or of the oligomer or polymer of the compound.
23. The use of para.21 or para. 22 wherein the compound is an imidazolium or an imidazolium salt.
24. The use of para. 21 or para. 22 wherein the compound is an imidazolinium or an imidazolinium salt.
25. The use of any one of paras. 21 to 24 wherein R⁵ is the same as R⁶.
26. The use of any one of paras. 21 to 25 wherein R⁵ and R⁶ are hydrocarbons.
27. The use of any one of paras. 21 to 26 wherein the pharmaceutically acceptable salt is a chloride, bromide, tetrafluoroborate or hexafluorophosphate salt.
28. The use of any one of paras. 21 to 27 wherein R⁷ is H.
29. The use of para. 21 or para. 22 wherein the compound is 1-ethyl-3-methylimidazolium, 1,3-bisbenzylimidazolium, 1,3-diisopropylimidazolium, 1,3-di-tert-butylimidazolinium, 1,3-bis(1-adamantyl)imidazolium, 1,3-bis(2,4,6-trimethylphenyl)-imidazalinium, 1,3-bis(2,6-diisopropyl-phenyl)-imidazolinium, 1,3,-bisbenzylbenzimidazolium, 1,3 -dibenzyl-2-methylimidazolium, 1,3-diallylimidazolium, 1-benzyl-3-methylimidazolium, 1-butyl-3-methylimidazolium, 1-(1-adamantyl)-3-(2,4,6-trimethylphenyl)-4,5-dihydroimidazolium, 2-benzylimidazo[1,5-a]quinolinium, 1,3-bis(1-adamantyl)-benzimidazolium, 1,3-dicyclohexylbenzimidazolium, 1,3-diisopropylimidazolinium tetrafluoroborate, 1,3-diisopropylimidazolium, 2-(2,6-diisopropylphenyl)-5-methylimidazo[1,5-α]pyridinium, 1-(2,6-diisopropylphenyl)-3-(2,4,6-trimethylphenyl)-imidazolinium, 2-mesityl-5-methylimidazo[1,5-α]pyridinium, 2-mesityl-2,5,6,7-tetrahydropyrrolo[2,1-*c*][1,2,4]triazol-4-ium, 1,3-bis(1-adamantyl)imidazolinium, 1-butyl-3-(2-pyridinylmethyl)-1*H*-imidazolium, 6,7-dihydro-2-pentafluorophneyl-5H-pyrrolo[2,1-*c*]-1,2,4-trizolium, 1-methyl-3-(2-hydroxylethyl)-imidazolium, 1-methyl-3-(4-isocynatobenzyl)-imidazolium, 1-methyl-3-(4-carboxylbenzyl)-imidazolium, 1-methyl-3-(4-acetate-benzyl)-imidazolium, 1-methyl-3-(2,2-dimethoxylethyl)-imidazolium, 1-(2,4,6-trimethylphenyl)-3-(4-acetate-benzyl)-imidazolium, 1,3-Dibenzyl-5-phenylimidazolium, 1-benzyl-3-(4-carboxylbenzyl)-2-methylimidazolium, 1-benzyl-3-(3,4,5-trimethoxylbenzyl)-2-methylimidazolium, 1-benzyl-3-(4-acetate-benzyl}-2-methyl-imidazolium, 1-benzyl-3-(4-methylcarboxylatebenzyl)-2-methyl-imidazolium, 1-benzyl-3-(2,2-dimethoxylethyl)-2-methyl-imidazolium, 2,5-di-(3-benzyl-imidazolium)-pyridine, 2,2'-di-(3-benzyl-imidazolium)-1,1'-binaphthalene, 1-benzyl-3-(4-methylbenzyl)-2-methylimidazolium, 1-benzyl-3-(2-trifluoromethylbenzyl)-2-methylimidazolium, 1-benzyl-3-(4-methylcarboxylatebenzyl)-5-phenylimidazolium, 1-benzyl-3-(4-acetatebenzyl)-5-phenyl-imidazolium, 1-benzyl-3-(4-methylbenzyl)-5-phenylimidazolium, (1,24,5-dimidazolium)-N,N',N",N'''-tetrabenzyl-benzene, 1-benzyl-3-(2-propyn-1-yl)-imidazolium, 1-benzyl-3-(3-hydroxyl-propyl)-imidazolium, 1,3-di(2-phenylethyl)-imidazolium, 1-benzyl-3-(4-acetatebenzyl)-imidazolium, 1-benzyl-3-(pyridin-2-yl)-imidazolium, 1,3,5-tris-(4-methyl-imidazolium)-linked cyclophane, 1,3-dibenzyl-2-(1,3-dibenzyl-1H-imidazol-2(3H)-ylidene)-2,3-dihydro-1H-imidazole, 1-benzyl-3-methylimidazolium, 1-(4-cyanatobenzyl)-3-methyl-imidazolium, 1-(4-carboxybenzyl)-3-methyl-imidazolium, 1-methyl-3-(4-acetate-benzyl)-imidazolium, 1-methyl-3-(2,2-dimethoxyethyl)-imidazolium, or 1-(2,4,6-trimethylphenyl)-3-(4-acetate-benzyl)-imidazolium, or a pharmaceutically acceptable salt thereof
30. The use of para. 21 or para. 22 wherein the compound is 1,3-bisbenzylimidazolium, 1,3-diisopropylimidazolium, 1,3-di-tert-butylimidazolinium, 1,3-bis(1-adamantyl)imidazolium, 1,3-bis(2,4,6-trimethylphenyl]-imidazolinium, 1,3-bis(2,6 -diisopropyl-phenyl)-imidazolinium, 1,3,-bisbenzylbenzimidazolium, or 1,3,5-tris-(4-methyl-imidazolium)-linked cyclophane, or a pharmaceutically acceptable salt thereof.
31. The use of para. 21 or para. 22 wherein the compound is 1,3-bisbenzylimidazolium or a pharmaceutically acceptable salt thereof.
32. The use of para.21 or para. 22 wherein the compound is 1,3-diisopropylimidazolium or a pharmaceutically acceptable salt thereof.
33. The use of para.21 or para. 22 wherein the compound is a dimer of a compound having a structure of general formula I or a pharmaceutically acceptable salt thereof.
34. The use of para.33 wherein the compound is 1,3-dibenzyl-2-(1,3-dibenzyl-1H-imidazol-2(3H)-ylidene)-2,3-dihydro-1H-imidazole, (1,2-4,5-diimidazolium)-N,N',N", N"'-tetrabenzyl-benzene, 2,6-di-(3-benzyl-imidazolium)-pyridine or 2,2'-di-(3-benzyl-imidazolium)-1,1'-binaphthalene, or a pharmaceutically acceptable salt thereof.
35. The use of para. 21 or para. 22 in which the compound is a trimer of a compound having a structure of general formula I or a pharmaceutically acceptable salt thereof.
36. The use of para. 35 wherein the compound is 1,3,5-tris(4-methyl-imidazolium)-linked cyclophane or a pharmaceutically acceptable salt thereof.
37. The use any one of paras. 21 to 36 wherein the use comprises use of an effective amount of the compound for treatment of a neurological disorder in a subject.
38. The use of para.37 wherein the neurological disorder is Parkinson's disease.
39. The use of para. 37 wherein the neurological disorder is retinopathy.

## Claims

1. An effective amount of a compound for use in the treatment of a neurological disorder in a subject, the compound being:
a) one of 1,3-di-tert-butylimidazolinium, 1,3-bis(1-adamantyl)imidazolium, 1,3-bis(2,4,6-trimethylphenyl)-imidazolinium, 1,3-bis(2,6-diisopropyl-phenyl)-imidazolinium, 1-(1-adamantyl)-3-(2,4,6-trimethylphenyl)-4,5-dihydroimidazolium, 2-benzylimidazo[1,5-a]quinolinium, 1,3-bis(1-adamantyl)-benzimidazolium, 1,3-diisopropylimidazolinium, 2-(2,6-diisopropylphenyl)-5-methylimidazo[1,5-a]pyridinium, 1-(2,6-diisopropylphenyl)-3-(2,4,6-trimethylphenyl)-imidazolinium, 2-mesityl-5- methylimidazo[1,5-a]pyridinium, 2-mesityl-2,5,6,7-tetrahydropyrrolo[2,1-c][1,2,4]triazol-4-ium, 1,3-bis(1-adamantyl)imidazolinium, 6,7-dihydro-2-pentafluorophneyl-5H-pyrrolo[2,1-c]-1,2,4-trizolium, 1-methyl-3-(2-hydroxylethyl)-imidazolium, 1-methyl-3-(4-isocynatobenzyl)-imidazolium, 1-methyl-3-(4-acetate-benzyl)-imidazolium, 1-methyl-3-(2,2-dimethoxylethyl)-imidazolium, 1-(2,4,6-trimethylphenyl)-3-(4-acetate-benzyl)-imidazolium, 1-benzyl-3-(4-acetate-benzyl)-2-methyl-imidazolium, 1-benzyl-3-(2,2-dimethoxylethyl)-2-methyl-imidazolium, 1-benzyl-3-(4-acetatebenzyl)-5-phenyl-imidazolium, 1-benzyl-3-(4-methylbenzyl)-5-phenylimidazolium, 1-benzyl-3-(3-hydroxyl-propyl)-imidazolium, 1-benzyl-3-(4-acetatebenzyl)-imidazolium, 1-(4-cyanatobenzyl)-3-methyl-imidazolium, 1-(4-carboxybenzyl)-3-methyl-imidazolium, 1,3-bis(2,6-diisopropylphenyl)imidazolium and 1,3-di-tert-butylimidazolium, or any dimer thereof;
or
b) 2,6-di-(3-benzyl-imidazolium)-pyridine, 2,2'-di-(3-benzyl-imidazolium)-1,1'-binaphthalene, (1,2-4,5-diimidazolium)-N,N',N",N"'-tetrabenzyl-benzene, 1,3,5-tris-(4-methyl-imiazolium)-linked cyclophane and 1,3-dibenzyl-2-(1,3-dibenzyl-1 H-imidazol-2(3H)-ylidene)-2,3-dihydro-1 H-imidazole;
or any pharmaceutically acceptable salt thereof.

2. The compound of claim 1 wherein the compound is 1,3-di-tert-butylimidazolinium, 1,3-bis(1-adamantyl)imidazolium, 1,3-bis(2,4,6-trimethylphenyl)-imidazolinium, 1,3-bis(2,6-diisopropyl-phenyl)-imidazolinium, 1-(1-adamantyl)-3-(2,4,6-trimethylphenyl)-4,5-dihydroimidazolium, 2-benzylimidazo[1,5-a]quinolinium, 1,3-bis(1-adamantyl)-benzimidazolium, 1,3-diisopropylimidazolinium, 2-(2,6-diisopropylphenyl)-5-methylimidazo[1,5-a]pyridinium, 1-(2,6-diisopropylphenyl)-3-(2,4,6-trimethylphenyl)-imidazolinium, 2-mesityl-5- methylimidazo[1,5-a]pyridinium, 2-mesityl-2,5,6,7-tetrahydropyrrolo[2,1-c][1,2,4]triazol-4-ium, 1,3-bis(1-adamantyl)imidazolinium, 6,7-dihydro-2-pentafluorophneyl-5H-pyrrolo[2,1-c]-1,2,4-trizolium, 1-methyl-3-(2-hydroxylethyl)-imidazolium, 1-methyl-3-(4-isocynatobenzyl)-imidazolium, 1-methyl-3-(4-acetate-benzyl)-imidazolium, 1-methyl-3-(2,2-dimethoxylethyl)-imidazolium, 1-(2,4,6-trimethylphenyl)-3-(4-acetate-benzyl)-imidazolium, 1-benzyl-3-(4-acetate-benzyl)-2-methylimidazolium, 1-benzyl-3-(2,2-dimethoxylethyl)-2-methyl-imidazolium, 1-benzyl-3-(4-acetatebenzyl)-5-phenyl-imidazolium, 1-benzyl-3-(4-methylbenzyl)-5-phenylimidazolium, 1-benzyl-3-(3-hydroxyl-propyl)-imidazolium, 1-benzyl-3-(4-acetatebenzyl)-imidazolium, 1-(4-cyanatobenzyl)-3-methyl-imidazolium, 1-(4-carboxybenzyl)-3-methyl-imidazolium, 1,3-bis(2,6-diisopropylphenyl)imidazolium or 1,3-di-tert-butylimidazolium, or any dimer thereof, or any pharmaceutically acceptable salt thereof.

3. The compound of claim 2 wherein the compound is 1,3-di-tert-butylimidazolinium, 1,3-bis(1-adamantyl)imidazolium, 1,3-bis(2,4,6-trimethylphenyl)-imidazolinium, or 1,3-bis(2,6-diisopropyl-phenyl)-imidazolinium, or any pharmaceutically acceptable salt thereof.

4. The compound of claim 1 wherein the compound is 2,6-di-(3-benzyl-imidazolium)-pyridine, 2,2'-di-(3-benzyl-imidazolium)-1,1'-binaphthalene, (1,2-4,5-diimidazolium)-N,N',N",N"'-tetrabenzyl-benzene, 1,3,5-tris-(4-methyl-imiazolium)-linked cyclophane and 1,3-dibenzyl-2-(1,3-dibenzyl-1H-imidazol-2(3H)-ylidene)-2,3-dihydro-1H-imidazole, or any pharmaceutically acceptable salt thereof.

5. The compound of claim 4 wherein the compound is 1,3,5-tris(4-methyl-imidazolium)-linked cyclophane or any pharmaceutically acceptable salt thereof.

6. The compound of any one of claims 1 to 5 wherein the pharmaceutically acceptable salt is a chloride, bromide, tetrafluoroborate or hexafluorophosphate salt.

7. The compound of any one of claims 1 to 6 wherein the neurological disorder is Parkinson's disease or retinopathy.

8. An *in vitro* method for delivering a neuroprotective agent to an *in vitro* neural cell, the method comprising contacting the neural cell with a compound that is
a) one of 1,3-di-tert-butylimidazolinium, 1,3-bis(1-adamantyl)imidazolium, 1,3-bis(2,4,6-trimethylphenyl)-imidazolinium, 1,3-bis(2,6-diisopropyl-phenyl)-imidazolinium, 1-(1-adamantyl)-3-(2,4,6-trimethylphenyl)-4,5-dihydroimidazolium, 2-benzylimidazo[1,5-a]quinolinium, 1,3-bis(1-adamantyl)-benzimidazolium, 1,3-diisopropylimidazolinium, 2-(2,6-diisopropylphenyl)-5-methylimidazo[1,5-a]pyridinium, 1-(2,6-diisopropylphenyl)-3-(2,4,6-trimethylphenyl)-imidazolinium, 2-mesityl-5- methylimidazo[1,5-a]pyridinium, 2-mesityl-2,5,6,7-tetrahydropyrrolo[2,1-c][1,2,4]triazol-4-ium, 1,3-bis(1-adamantyl)imidazolinium, 6,7-dihydro-2-pentafluorophneyl-5H-pyrrolo[2,1-c]-1,2,4-trizolium, 1-methyl-3-(2-hydroxylethyl)-imidazolium, 1-methyl-3-(4-isocynatobenzyl)-imidazolium, 1-methyl-3-(4-acetate-benzyl)-imidazolium, 1-methyl-3-(2,2-dimethoxylethyl)-imidazolium, 1-(2,4,6-trimethylphenyl)-3-(4-acetate-benzyl)-imidazolium, 1-benzyl-3-(4-acetate-benzyl)-2-methyl-imidazolium, 1-benzyl-3-(2,2-dimethoxylethyl)-2-methyl-imidazolium, 1-benzyl-3-(4-acetatebenzyl)-5-phenyl-imidazolium, 1-benzyl-3-(4-methylbenzyl)-5-phenylimidazolium, 1-benzyl-3-(3-hydroxyl-propyl)-imidazolium, 1-benzyl-3-(4-acetatebenzyl)-imidazolium, 1-(4-cyanatobenzyl)-3-methyl-imidazolium, 1-(4-carboxybenzyl)-3-methyl-imidazolium, 1,3-bis(2,6-diisopropylphenyl)imidazolium and 1,3-di-tert-butylimidazolium, or any dimer thereof;
or
b) 2,6-di-(3-benzyl-imidazolium)-pyridine, 2,2'-di-(3-benzyl-imidazolium)-1,1'-binaphthalene, (1,2-4,5-diimidazolium)-N,N',N",N"'-tetrabenzyl-benzene, 1,3,5-tris-(4-methyl-imiazolium)-linked cyclophane and 1,3-dibenzyl-2-(1,3-dibenzyl-1H-imidazol-2(3H)-ylidene)-2,3-dihydro-1 H-imidazole;
or any pharmaceutically acceptable salt thereof.

9. The method of claim 8 wherein the compound is 1,3-di-tert-butylimidazolinium, 1,3-bis(1-adamantyl)imidazolium, 1,3-bis(2,4,6-trimethylphenyl)-imidazolinium, 1,3-bis(2,6-diisopropylphenyl)-imidazolinium, 1-(1-adamantyl)-3-(2,4,6-trimethylphenyl)-4,5-dihydroimidazolium, 2-benzylimidazo[1,5-a]quinolinium, 1,3-bis(1-adamantyl)-benzimidazolium, 1,3-diisopropylimidazolinium, 2-(2,6-diisopropylphenyl)-5-methylimidazo[1,5-a]pyridinium, 1-(2,6-diisopropylphenyl)-3-(2,4,6-trimethylphenyl)-imidazolinium, 2-mesityl-5- methylimidazo[1,5-a]pyridinium, 2-mesityl-2,5,6,7-tetrahydropyrrolo[2,1-c][1,2,4]triazol-4-ium, 1,3-bis(1-adamantyl)imidazolinium, 6,7-dihydro-2-pentafluorophneyl-5H-pyrrolo[2,1-c]-1,2,4-trizolium, 1-methyl-3-(2-hydroxylethyl)-imidazolium, 1-methyl-3-(4-isocynatobenzyl)-imidazolium, 1-methyl-3-(4-acetate-benzyl)-imidazolium, 1-methyl-3-(2,2-dimethoxylethyl)-imidazolium, 1-(2,4,6-trimethylphenyl)-3-(4-acetate-benzyl)-imidazolium, 1-benzyl-3-(4-acetate-benzyl)-2-methylimidazolium, 1-benzyl-3-(2,2-dimethoxylethyl)-2-methyl-imidazolium, 1-benzyl-3-(4-acetatebenzyl)-5-phenyl-imidazolium, 1-benzyl-3-(4-methylbenzyl)-5-phenylimidazolium, 1-benzyl-3-(3-hydroxyl-propyl)-imidazolium, 1-benzyl-3-(4-acetatebenzyl)-imidazolium, 1-(4-cyanatobenzyl)-3-methyl-imidazolium, 1-(4-carboxybenzyl)-3-methyl-imidazolium, 1,3-bis(2,6-diisopropylphenyl)imidazolium or 1,3-di-tert-butylimidazolium, or any dimer thereof, or any pharmaceutically acceptable salt thereof.

10. The method of claim 9 wherein the compound is 1,3-di-tert-butylimidazolinium, 1,3-bis(1-adamantyl)imidazolium, 1,3-bis(2,4,6-trimethylphenyl)-imidazolinium, or 1,3-bis(2,6-diisopropylphenyl)-imidazolinium, or any pharmaceutically acceptable salt thereof.

11. The method of claim 10 wherein the compound is 2,6-di-(3-benzyl-imidazolium)-pyridine, 2,2'-di-(3-benzyl-imidazolium)-1,1'-binaphthalene, (1,2-4,5-diimidazolium)-N,N',N",N"'-tetrabenzyl-benzene, 1,3,5-tris-(4-methyl-imiazolium)-linked cyclophane and 1,3-dibenzyl-2-(1,3-dibenzyl-1H-imidazol-2(3H)-ylidene)-2,3-dihydro-1H-imidazole, or any pharmaceutically acceptable salt thereof.

12. The method of claim 11 wherein the compound is 1,3,5-tris(4-methyl-imidazolium)-linked cyclophane or any pharmaceutically acceptable salt thereof.

13. The method of any one of claims 8 to 12 wherein the pharmaceutically acceptable salt is a chloride, bromide, tetrafluoroborate or hexafluorophosphate salt.
